(19) Europäisches Patentamt
European Patent Office
Office européen des brevets

(11) Veröffentlichungsnummer : **0 276 196 B1**

(12) # EUROPÄISCHE PATENTSCHRIFT

(45) Veröffentlichungstag der Patentschrift : **18.09.91 Patentblatt 91/38**

(21) Anmeldenummer : **88810011.2**

(22) Anmeldetag : **13.01.88**

(51) Int. Cl.[5] : **C07D 317/14,** C07D 317/72, C07D 317/46, C07D 409/04, C07D 319/12, C07D 319/14, C07D 405/12, A01N 43/28, A01N 43/30, A01N 43/32

(54) **Substituierte Dioxolanderivate.**

Teilanmeldung 90117463.1 eingereicht am 13/01/88.
Die Akte enthält technische Angaben, die nach dem Eingang der Anmeldung eingereicht wurden und die nicht in dieser Patentschrift enthalten sind.

(30) Priorität : **19.01.87 CH 177/87**

(43) Veröffentlichungstag der Anmeldung : **27.07.88 Patentblatt 88/30**

(45) Bekanntmachung des Hinweises auf die Patenterteilung : **18.09.91 Patentblatt 91/38**

(84) Benannte Vertragsstaaten : **AT BE CH DE ES FR GB GR IT LI NL**

(56) Entgegenhaltungen :
**US-A- 4 007 280**
**US-A- 4 097 581**
**US-A- 4 590 282**

(73) Patentinhaber : **CIBA-GEIGY AG**
**Klybeckstrasse 141**
**CH-4002 Basel (CH)**

(72) Erfinder : **Karrer, Friedrich, Dr.**
**Rebbergstrasse 5**
**CH-4800 Zofingen (CH)**

EP 0 276 196 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte 1,3-Dioxolanderivate, Verfahren zu ihrer Herstellung und ihre Verwendung in der Schädlingsbekämpfung.

Die erfindungsgemässen Verbindungen haben die Formel I

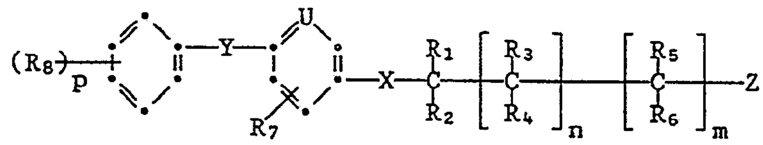

(I),

worin

$R_1$, $R_2$, $R_3$, $R_4$, $R_5$ und $R_6$ unabhängig voneinander Wasserstoff oder $C_1$-$C_4$-Alkyl oder, wenn n = 1,

$R_2$ und $R_4$ zusammen einen der Reste $-(CH_2)_3-$ oder $-(CH_2)_4-$ ;

$R_7$ Wasserstoff, Halogen, Methyl, Aethyl, mit 1 bis 5 Halogenatomen substituiertes $C_1$-$C_2$-Alkyl, Methoxy, Aethoxy oder mit 1 bis 5 Halogenatomen substituiertes $C_1$-$C_2$-Alkoxy;

$R_8$ Halogen, $C_1$-$C_3$-Alkyl, mit 1 bis 7 Halogenatomen substituiertes $C_1$-$C_3$-Alkyl, $C_1$-$C_3$-Alkoxy, mit 1 bis 7 Halogenatomen substituiertes $C_1$-$C_3$-Alkoxy oder Cyano;

U eine Gruppierung -CH= oder -N= ;

X -O- , -S- oder -N($R_9$)- ;

Y -O- , -S- , -S(O)- , -S($O_2$)- , -$CH_2$- , -CO- oder -N($R_9$)- , wobei

$R_9$ für Wasserstoff, $C_1$-$C_4$-Alkyl oder Alkylcarbonyl mit insgesamt 2 bis 4 C-Atomen steht;

n eine Zahl 0 oder 1;

m eine Zahl 0, 1 oder 2;

p eine Zahl 1, 2 oder 3; und

Z den Rest

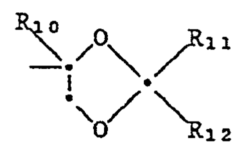

bedeuten, wobei

$R_{10}$ Wasserstoff, Methyl oder Aethyl;

$R_{11}$ Wasserstoff, $C_1$-$C_6$-Alkyl, $C_3$-$C_6$-Cycloalkyl, $C_2$-$C_4$-Alkenyl, $C_2$-$C_4$-Alkinyl, Alkoxyalkyl mit insgesamt 2 bis 6 C-Atomen, mit 1 bis 7 Halogenatomen substituiertes $C_1$-$C_6$-Alkyl, Benzyl, oder durch ein Halogenatom, einen $C_1$-$C_3$-Alkylrest oder $C_1$-$C_3$-Alkoxyrest kernsubstituiertes Benzyl, Cyanomethyl, ($C_1$-$C_2$-Alkoxy)-Carbonyl oder den Rest -$CH_2$-NH-COO-($C_1$-$C_2$-Alkyl); und

$R_{12}$ Wasserstoff, $C_1$-$C_6$-Alkyl, Phenyl, mit 1 bis 3 Substituenten aus der Gruppe Halogen, $C_1$-$C_4$-Alkyl, mit 1 bis 7 Halogenatomen substituiertes $C_1$-$C_3$-Alkyl, $C_2$-$C_4$-Alkenyl, $C_2$-$C_4$-Alkinyl, $C_1$-$C_3$-Alkoxy, mit 1 bis 7 Halogenatomen substituiertes $C_1$-$C_3$-Alkoxy, Alkoxyalkyl mit insgesamt 2 bis 5 C-Atomen, $C_3$-$C_4$-Alkenyloxy, $C_3$-$C_4$-Alkinyloxy und $C_1$-$C_3$-Alkylthio substituiertes Phenyl, Pyridyl, Furan-2-yl, Thien-2-yl oder den Rest

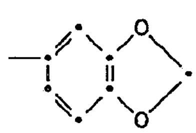

;

oder

$R_{12}$ und $R_{11}$ zusammen eine der Gruppen $-(CH_2)_3-$ , $-(CH_2)_4-$ oder $-(CH_2)_5-$ bedeuten.

Halogen steht im Rahmen der vorliegenden Erfindung für Fluor, Chlor, Brom oder Jod, vorzugsweise für Fluor oder Chlor.

Der Erfindungsgegenstand umfasst auch die möglichen Isomeren, einschliesslich der Diastereomeren und der Enantiomeren, der Verbindungen der Formel I.

Die Alkyl-, Alkoxy-, Halogenalkyl-, Halogenalkoxy-, Alkoxyalkyl, Alkylthioalkyl-, Alkenyl- und Alkinylgruppen können geradkettig oder verzweigt sein. Beispiele solcher Gruppen sind u.a. Methyl, Methoxy, Methoxymethyl, Difluormethoxy, Aethyl, Aethoxy, 2-Fluoräthoxy, 1,1,2,2-Tetrafluoräthyl, Pentafluoräthyl, 1,1,2,2-Tetrafluoräthoxy, Pentafluoräthoxy, Propyl, Isopropyl, n-Butyl, n-Pentyl, n-Hexyl und deren Isomere, Vinyl, 1-Propen-3-yl, 1-Propinyl.

Bevorzugte Cycloalkylgruppen bei $R_{11}$ sind Cyclopentyl und Cyclohexyl.

Bevorzugt sind erfindungsgemäss Verbindungen der Formel I, worin

$R_1$, $R_2$, $R_3$, $R_4$, $R_5$ und $R_6$ unabhängig voneinander Wasserstoff oder $C_1$-$C_4$-Alkyl oder, wenn n = 1,

$R_2$ und $R_4$ zusammen einen der Reste $-(CH_2)_3-$ oder $-(CH_2)_4-$ ;

$R_7$ Wasserstoff, Halogen, Methyl, Aethyl, mit 1 bis 5 Halogenatomen substituiertes $C_1$-$C_2$-Alkyl, Methoxy, Aethoxy oder mit 1 bis 5 Halogenatomen substituiertes $C_1$-$C_2$-Alkoxy;

$R_8$ Halogen, $C_1$-$C_3$-Alkyl, mit 1 bis 7 Halogenatomen substituiertes $C_1$-$C_3$-Alkyl, $C_1$-$C_3$-Alkoxy, mit 1 bis 7 Halogenatomen substituiertes $C_1$-$C_3$-Alkoxy oder Cyano;

U eine Gruppierung -CH= oder -N= ;

X -O- , -S- oder -N($R_9$)- ;

Y -O- , -S- , -S(O)- , -S($O_2$)- , -$CH_2$- , -CO- oder -N($R_9$)- , wobei

$R_9$ für Wasserstoff, $C_1$-$C_4$-Alkyl oder Alkylcarbonyl mit insgesamt 2 bis 4 C-Atomen steht;

n eine Zahl 0 oder 1;

m eine Zahl 0, 1 oder 2;

p eine Zahl 1, 2 oder 3; und

Z den Rest

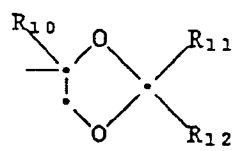

bedeuten, wobei

$R_{10}$ Wasserstoff, Methyl oder Aethyl;

$R_{11}$ Wasserstoff, $C_1$-$C_6$-Alkyl, $C_3$-$C_6$-Cycloalkyl, $C_2$-$C_4$-Alkenyl, $C_2$-$C_4$-Alkinyl, Alkoxyalkyl mit insgesamt 2 bis 6 C-Atomen, mit 1 bis 7 Halogenatomen substituiertes $C_1$-$C_6$-Alkyl, Benzyl, oder durch ein Halogenatom, einen $C_1$-$C_3$-Alkylrest oder $C_1$-$C_3$-Alkoxyrest kernsubstituiertes Benzyl; und

$R_{12}$ Wasserstoff, $C_1$-$C_6$-Alkyl, Phenyl, mit 1 bis 3 Substituenten aus der Gruppe Halogen, $C_1$-$C_4$-Alkyl, mit 1 bis 7 Halogenatomen substituiertes $C_1$-$C_3$-Alkyl, $C_2$-$C_4$-Alkenyl, $C_2$-$C_4$-Alkinyl, $C_1$-$C_3$-Alkoxy, mit 1 bis 7 Halogenatomen substituiertes $C_1$-$C_3$-Alkoxy, Alkoxyalkyl mit insgesamt 2 bis 5 C-Atomen, $C_3$-$C_4$-Alkenyloxy, $C_3$-$C_4$-Alkinyloxy und $C_1$-$C_3$-Alkylthio substituiertes Phenyl oder den Rest

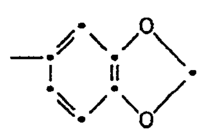 ;

oder

$R_{12}$ und $R_{11}$ zusammen einen der Reste $-(CH_2)_4-$ oder $-(CH_2)_5-$ bedeuten.

Hervorzuheben sind auch Verbindungen der Formel I, die dadurch gekennzeichnet sind, dass

$R_1$ und $R_3$ unabhängig voneinander Wasserstoff, Methyl oder Aethyl;

$R_2$, $R_4$, $R_5$ und $R_6$ Wasserstoff oder, wenn n = 1,

$R_2$ und $R_4$ zusammen einen der Reste $-(CH_2)_3-$ oder $-(CH_2)_4-$ ;

$R_7$ Wasserstoff oder Halogen;

$R_8$ Methyl, Aethyl, Halogen oder mit 1 bis 5 Halogenatomen substituiertes $C_1$-$C_2$-Alkyl;

U eine Gruppierung -CH= oder -N= ;
X -O- , -S- oder -N($R_9$)- ;
Y -O- , -S- , -$CH_2$- oder -CO- ; wobei
$R_9$ für Wasserstoff, Methyl oder Acetyl steht;
n und m unabhängig voneinander eine Zahl 0 oder 1; und
p eine Zahl 1 oder 2; und
Z den Rest

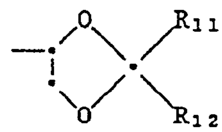

bedeuten, wobei
$R_{11}$ Wasserstoff, $C_1$-$C_4$-Alkyl, $C_3$-$C_6$-Cycloalkyl, $C_2$-$C_3$-Alkenyl, $C_2$-$C_3$-Alkinyl, Alkoxyalkyl mit insgesamt 2 bis 4 C-Atomen, mit 1 bis 7 Halogenatomen substituiertes $C_1$-$C_4$-Alkyl oder Benzyl; und
$R_{12}$ Wasserstoff, $C_1$-$C_4$-Alkyl, Phenyl, mit 1 oder 2 Substituenten aus der Gruppe Halogen, $C_1$-$C_3$-Alkyl, mit 1 bis 5 Halogenatomen substituiertes $C_1$-$C_2$-Alkyl, $C_2$-$C_4$-Alkenyl, $C_2$-$C_4$-Alkinyl, $C_1$-$C_3$-Alkoxy, mit 1 bis 3 Halogenatomen substituiertes Methoxy, Alkoxyalkyl mit insgesamt 3 bis 4 C-Atomen, $C_3$-$C_4$-Alkenyloxy, $C_3$-$C_4$-Alkinyloxy und $C_1$-$C_3$-Alkylthio substituiertes Phenyl oder den Rest

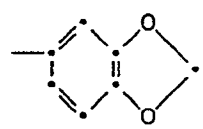 ;

oder

$R_{12}$ und $R_{11}$ zusammen einen der Reste $-(CH_2)_4-$ oder $-(CH_2)_5-$ bedeuten.
Weiterhin bevorzugt sind diejenigen Verbindungen der Formel I, worin
$R_1$ Wasserstoff oder Methyl;
$R_2$, $R_3$, $R_4$, $R_5$, $R_6$ und $R_7$ Wasserstoff;
$R_8$ Fluor, Chlor, Methyl oder Trifluormethyl;
U die Gruppierung -CH= ;
X -O- ;
Y -O- ;
n und m die Zahl 0;
p eine Zahl 1 oder 2; und
Z den Rest

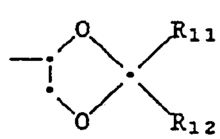

bedeuten, wobei
$R_{11}$ Wasserstoff, $C_1$-$C_4$-Alkyl, $C_3$-$C_6$-Cycloalkyl, $C_2$-$C_3$-Alkenyl, $C_2$-$C_3$-Alkinyl, Alkoxyalkyl mit insgesamt 2 bis 4 C-Atomen oder Benzyl;
$R_{12}$ Wasserstoff, $C_1$-$C_4$-Alkyl, mit 1 bis 5 Halogenatomen substituiertes $C_1$-$C_2$-Alkyl, Phenyl, mit 1 oder 2 Substituenten aus der Gruppe Halogen und $C_1$-$C_3$-Alkyl, substituiertes Phenyl oder den Rest

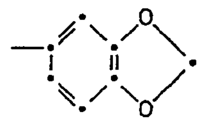 ;

oder

$R_{12}$ und $R_{11}$ zusammen einen der Reste $-(CH_2)_4-$ oder $-(CH_2)_5-$ bedeuten;
diejenigen Verbindungen der Formel I, worin
$R_1$ Wasserstoff oder Methyl;
$R_2$, $R_3$, $R_4$, $R_5$, $R_6$ und $R_7$ Wasserstoff
$R_8$ Halogen oder Trifluormethyl;
U die Gruppierung -CH=;
X -O- oder -S-;
Y -O- , -S- , $-CH_2-$ oder -CO-;
n und m die Zahl 0;
p eine Zahl 1 oder 2; und
Z den Rest

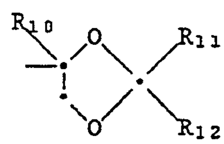

bedeuten, wobei
$R_{10}$ Wasserstoff oder Methyl;
$R_{11}$ Wasserstoff, $C_1$-$C_6$-Alkyl, $C_3$-$C_6$-Cycloalkyl, $C_2$-$C_4$-Alkenyl, $C_2$-$C_4$-Alkinyl, Alkoxyalkyl mit insgesamt 2 bis 4 C-Atomen, mit 1 bis 5 Halogenatomen substituiertes $C_1$-$C_4$-Alkyl, Benzyl, Cyanomethyl, ($C_1$-$C_2$-Alkoxy)-carbonyl oder den Rest $-CH_2$-NH-COO-($C_1$-$C_2$-Alkyl); und
$R_{12}$ Wasserstoff, $C_1$-$C_6$-Alkyl, mit 1 bis 2 Substituenten aus der Gruppe Halogen, $C_1$-$C_2$-Alkyl, $C_2$-$C_4$-Alkenyl, $C_2$-$C_3$-Alkinyl, $C_1$-$C_2$-Alkoxy, und $C_2$-$C_3$-Alkylthio substituiertes Phenyl, Pyridyl, Furan-2-yl, Thien-2-yl oder den Rest

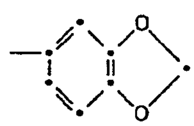 ;

oder

$R_{12}$ und $R_{11}$ zusammen eine der Gruppen $-(CH_2)_3-$, $-(CH_2)_4-$ oder $-(CH_2)_5-$ bedeuten;
sowie diejenigen Verbindungen der Formel I, worin
$R_1$, $R_2$, $R_3$, $R_4$, $R_5$, $R_6$ und $R_7$ Wasserstoff;
$R_8$ Fluor, Chlor oder Trifluormethyl;
U die Gruppierung -CH=;
X -O- ,
Y -O- , -S- , $-CH_2-$ oder -CO- ;
n und m die Zahl 0;
p eine Zahl 1 oder 2; und
Z den Rest

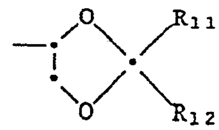

bedeuten, wobei

$R_{11}$ Wasserstoff, $C_1$-$C_6$-Alkyl, $C_3$-$C_6$-Cycloalkyl, $C_2$-$C_4$-Alkenyl, $C_2$-$C_4$-Alkinyl, Alkoxyalkyl mit insgesamt 2 bis 4 C-Atomen, mit 1 bis 5 Fluor- oder Chlor-atomen substituiertes $C_1$-$C_4$-Alkyl, Benzyl, Cyanomethyl, Aethoxycarbonyl oder den Rest $-CH_2-NH-COO-CH_3$; und

$R_{12}$ Wasserstoff, $C_1$-$C_6$-Alkyl, mit 1 bis 2 Substituenten aus der Gruppe Fluor, Chlor, $C_1$-$C_2$-Alkyl, und $C_1$-$C_2$-Alkoxy, substituiertes Phenyl, Pyrid-4-yl, Furan-2-yl, oder Thien-2-yl oder den Rest

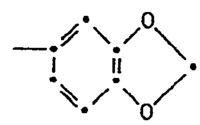 ;

oder

$R_{12}$ und $R_{11}$ zusammen eine der Gruppen $-(CH_2)_3-$, $-(CH_2)_4-$ oder $-(CH_2)_5-$ bedeuten.

Speziell hervorzuheben sind wegen ihrer biologischen Wirksamkeit die erfindungsgemässen Verbindungen der Formel Ia

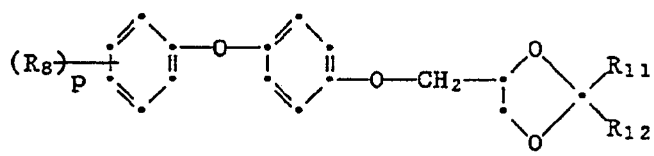 (Ia),

worin

$R_8$ Fluor, Chlor oder Trifluormethyl;

p eine Zahl 1 oder 2; und

$R_{11}$ und $R_{12}$ unabhängig voneinander Wasserstoff, $C_1$-$C_4$-Alkyl, $C_2$-$C_4$-Methoxyalkyl, mit 1 bis 3 Halogenatomen substituiertes $C_1$-$C_2$-Alkyl, $C_2$-$C_3$-Alkenyl oder $C_2$-$C_3$-Alkinyl oder $R_{11}$ und $R_{12}$ zusammen einen der Reste $-(CH_2)_4-$ oder $-(CH_2)_5-$ bedeuten.

Besondere Bedeutung kommt auch denjenigen Verbindungen der Formel I bzw. Ia zu, in denen p = 1 ist und/oder $R_{12}$ Wasserstoff bedeutet.

Die Verbindungen der Formel I bzw. Ia) können in an sich bekannter Weise hergestellt werden (vgl. auch US-Patentschrift No. 4,007,280):

a) Man kann hierzu z.B. eine Verbindung der Formel II

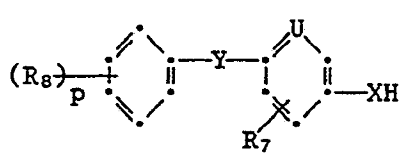 (II)

mit einer Verbindung der Formel III

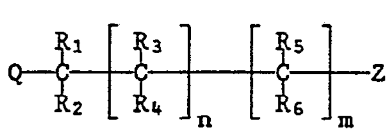 (III)

umsetzen, wobei in den Formeln II und III $R_1$ bis $R_8$, U, X, Y, n, m, p und Z die vorstehend angegebenen Bedeutungen haben und Q für eine Abgangsgruppe steht.

b) Eine Verbindung der Formel I, worin Z den Rest

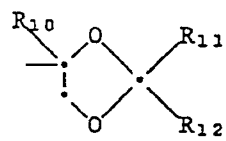

bedeutet, kann erhalten werden, indem man eine Verbindung der Formel IV

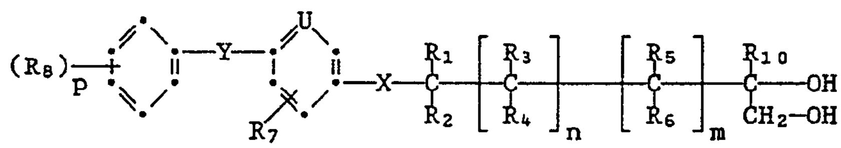

(IV)

in Gegenwart eines sauren Katalysators mit einer Verbindung der Formel V oder Va

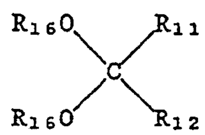

(V)

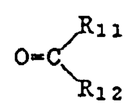

(Va)

kondensiert, wobei in den Formeln IV, V und Va $R_1$ bis $R_{10}$, U, X, Y, n, m, p, $R_{11}$ und $R_{12}$ die vorstehend angegebenen Bedeutungen haben und $R_{16}$ für $C_1$-$C_4$-Alkyl, bevorzugt Methyl oder Aethyl, steht.

Die Durchführung des Verfahrens a) erfolgt vorzugsweise in Gegenwart eines gegenüber den Umsetzungsteilnehmern inerten Lösungs- oder Verdünnungsmittels und mindestens eines Aequivalents eines Säureacceptors bzw. einer basischen Substanz. Als Säureacceptoren, bzw. Basen kommen insbesondere tertiäre Amine, wie Trialkylamine und Pyridin, ferner Hydride, Hydroxide, Oxide, Carbonate und Hydrogencarbonate von Alkali- und Erdalkalimetallen sowie auch Alkalimetallalkoholate, wie z.B. Kalium-t-butylat und Natriummethylat usw., in Betracht. Die Reaktionstemperatur liegt je nach der Art der verwendeten Lösungsmittel üblicherweise im Bereich von -5°C bis +140°C, vorzugsweise zwischen 0 und 60°C. Bei der Ausgangsverbindung der Formel III steht Q für eine herkömmliche Abgangsgruppe, wie z.B. ein Halogenatom die Mesyloxy- oder Tosyloxy-Gruppe.

Das Verfahren b) wird im allgemeinen in Gegenwart eines inerten Lösungs- oder Verdünnungsmittels und eines sauren Katalysators durchgeführt. Als saure Katalysatoren werden z.B. Sulfonsäuren, wie Methan- oder p-Toluolsulfonsäure, einschliesslich der sauren Ionenaustauscherharze mit Sulfogruppen, Lewis-Säuren, wie Bortrifluorid-Diäthyläther- oder -Dimethyläther-Komplexe, sowie Mineralsäuren, wie Schwefelsäure oder Phosphorsäure, verwendet. Die Reaktionstemperatur liegt je nach Art der verwendeten Lösungsmittel im Bereich von etwa 30 bis 150°C, vorzugsweise zwischen 60 und 120°C.

Die vorstehend aufgeführten Verfahren werden bevorzugt bei normalem oder erhöhtem Druck und vorzugsweise in einem inerten Lösungs- oder Verdünnungsmittel durchgeführt. Als Lösungs- oder Verdünnungsmittel für Verfahren a) eignen sich z.B. Aether und ätherartige Verbindungen, wie Diäthyläther, Diisopropyläther, Dioxan, 1,2-Dimethoxyäthan und Tetrahydrofuran; Amide, wie N,N-dialkylierte Carbonsäureamide; Sulfolan und Dimethylsulfoxid. Als Lösungs- oder Verdünnungsmittel für Verfahren b) eignen sich z.B. aliphatische, aromatische sowie halogenierte Kohlenwasserstoffe, insbesondere Benzol, Toluol, Xylole, Chloroform, Dichlormethan, Chlorbenzol, Hexan oder Cyclohexan.

Die Ausgangsstoffe der Formeln II bis Va sind bekannt oder können, falls sie neu sind, analog bekannten Methoden hergestellt werden. Sie bilden dann ebenfalls einen Gegenstand der vorliegenden Erfindung.

So können die Diole der Formel IV erhalten werden, indem man eine Verbindung der Formel II mit einem Hydroxymethylalkylenoxyd der Formel VII umsetzt (vgl. US-Patentschriften Nr. 4.590.282 und 4.097.581)

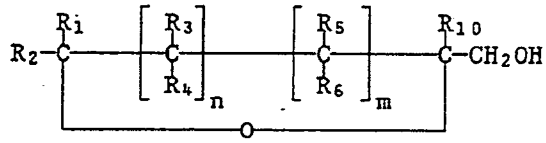

(VII)

Die Verbindungen der Formel I bzw. Ia) können als Gemische der verschiedenen enantiomeren Formen vorliegen, bzw. bei der Synthese gebildet werden. Die Diastereomerengemische bzw. Racemate der Formel I bzw. Ia können nach bekannten Methoden in die einzelnen Formen aufgetrennt werden. Unter der Verbindung der Formel I versteht man daher sinngemäss sowohl die einzelnen diastereoisomeren resp. enantiomeren Formen, als auch deren Gemische.

Aus der US-PS 4.097.581 sind bereits 4-(4-Phenoxy)-phenoxymethyl-1,3-dioxolane als Insektizide bekannt. Aehnlich strukturierte 1,3-Dioxolan- und 1,4-Benzodioxin-Derivate sowie deren Anwendung als Pestizide sind Gegenstand von US-PS 4.590.282. Demgegenüber unterscheiden sich die erfindungsgemässen Verbindungen der Formel I im wesentlichen durch das Vorliegen einer obligat mit 1 bis 3 Resten $R_8$ substituierten Phenylgruppe.

Es wurde überraschenderweise gefunden, dass sich die erfindungsgemässen Verbindungen der Formel I ausgezeichnet zur Bekämpfung von verschiedenartigen Schädlingen an Tieren und Pflanzen sowie im Boden eignen. So können sie zur Bekämpfung von Insekten, z.B. der Ordnung Lepidoptera, Coleoptera, Homoptera, Heteroptera, Diptera, Thysano-ptera, Orthoptera, Anoplura, Siphonaptera, Mallophaga, Thysanura, Isoptera, Psocoptera und Hymenoptera und von Milben und Zecken der Ordnung Acarina eingesetzt werden.

So eignen sich Verbindungen der Formel I bzw. Ia) zur Bekämpfung von pflanzenschädigenden Insekten in Zier- und Nutzpflanzungen, insbesondere in Baumwollkulturen (z.B. Spodoptera littoralis und Heliothis virescens). Die Verbindungen der Formel I haben ferner eine günstige Wirkung gegen Bodeninsekten (z.B. Aulacophora femoralis, Chortophila brassicae, Diabrotica balteata, Pachnoda savignyi und Scotia ypsilon) und insbesondere gegen saugende Insekten, wie Aphide (z.B. Myzus persicae, Aphis craccivora und Aonidiella aurantii sowie weitere Schildlausarten). Die erfindungsgemässen Verbindungen sind auch wirksam als Ovizide im Pflanzenschutz, speziell zur Bekämpfung von pflanzenschädigenden Insekten, wie z.B. Cydia pomonella, Lobesia botrana und Adoxophyes reticulana.

Wirkstoffe der Formel I zeigen auch eine sehr günstige Wirkung gegen Fliegen, wie z.B. Musca domestica und Mückenlarven sowie gegen Vorratsschädlinge, wie z.B. Sitophilus orizae und Rhizopertha dominica.

Die erfindungsgemässen Verbindungen der Formeln I und Ia) weisen eine besonders ausgeprägte Wirkung gegen pflanzenschädigende Akariden (Spinnmilben: z.B. der Familien Tetranychidae, Tarsonemidae, Eriophydae, Tyroglyphidae und Glycyphagidae) und auch gegen ektoparasitäre Akariden (Milben und Zecken: z.B. der Familien Ixodidae, Argasidae, Sacroptidae und Dermanyssidae), welche Nutztiere befallen, auf. Einige der erfindungsgemässen Verbindungen zeichnen sich durch gute akarizid-ovizide Aktivität und Blattpenetrationswirkung aus. Die erfindungsgemässen Verbindungen eignen sich vor allem zur Bekämpfung der folgenden, Obst- und Gemüsekulturen befallenden Milbenspezies: Tetranychus urticae, Tetranychus cinnabarinus, Panonychus ulmi, Bryobia rubrioculus, Panonychus citri, Eriophyes pyri, Eriophyes ribis, Eriophyes vitis, Tarsomemus pallidus, Phyllocoptes vitis und Phyllocoptura oleivora.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; pyrethrinartige Verbindungen sowie Karbamate und chlorierte Kohlenwasserstoffe.

Die gute pestizide Wirkung der erfindungsgemässen Verbindungen der Formeln I und Ia entspricht einer Abtötungsrate (Mortalität) von mindestens 50-60 % der erwähnten Schädlinge.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet.

Die Anwendungsverfahren wie Versprühen, Vernebeln, Verstäuben, Verstreuen oder Giessen werden gleich wie die Art der Mittel den angestrebten Zielen und den gegebenen Verhältnis-sen entsprechend gewählt.

Die Formulierungen, d.h. die mindenstens einen Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyl-äther, Essigester, Propylmyristat oder Propylpalmitat, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; Silikonöle oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5-Aethylenglykoleinheiten.

Als Beispiele nichtionischer Tenside seien Nonylphenolpolyäthoxyäthanole, Ricinusölpolyglycoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"McCutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides, wobei sich die %-Angaben auf das Gewicht beziehen.

Während als Handelsware eher konzentrierte Mittel bevorzugt werden, verwendet der Endverbraucher in der Regel verdünnte Mittel bzw. Zubereitungen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1:

a) Herstellung von 1,2-Dihydroxy-3-[4-(4-fluorphenoxy)]-phenoxypropan (Ausgangsprodukt):

Zu einer Lösung von 71,4 g 4-Fluorphenoxyphenol in 70 ml Xylol gibt man 0,7 g Tetramethylammoniumchlorid, erwärmt auf 60°C und tropft unter Rühren in ca. 30 Minuten, 29,6 g Glyceringlycid hinzu. Anschliessend wird das Reaktionsgemisch während etwa 16 Stunden bei 90°C nachgerührt. Zur Aufarbeitung wird das Reaktionsgemisch langsam bei ca. 60°C mit 200 ml n-Hexan versetzt, unter Rühren auf 20°C abgekühlt und der gebildete Niederschlag abfiltriert. Das so erhaltene Rohprodukt wird aus Isopropanol und n-Hexan umkristallisiert. Auf diese Weise erhält man die Titelverbindung der Formel

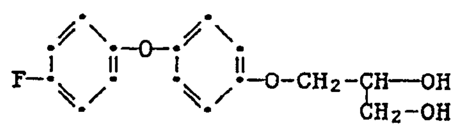

als farblose Kristalle mit einem Schmelzpunkt von 86-87°C.

In analoger Weise und gegebenenfalls mit chromatographischer Reinigung (Eluierungsmittel: Methylenchlorid/Diäthyläther 1:1) werden die folgenden Verbindungen der Formel IV hergestellt:

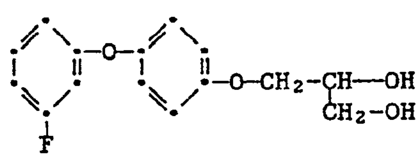

Smp. 76-78°C

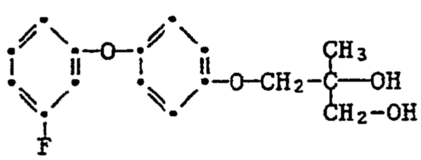

Smp. 63-64°C

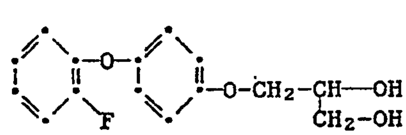

Smp. 52-54°C

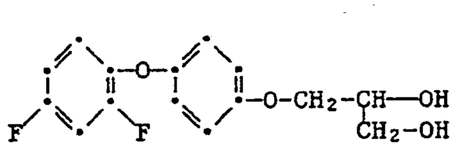

Smp. 64-65°C

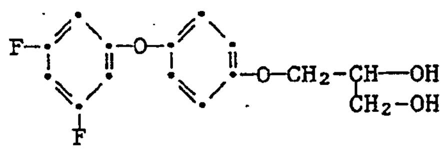

Smp. 74-75°C

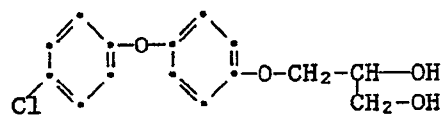

Smp. 109-110°C

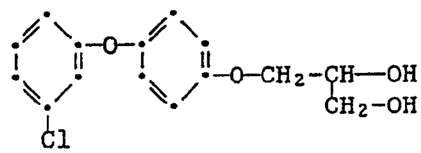

Smp. 60-62°C

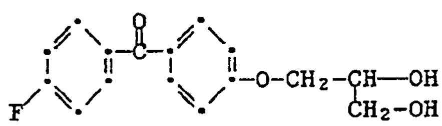

Smp. 94-96°C

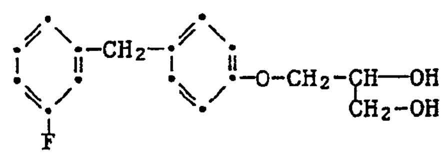

Smp. 81-82°C

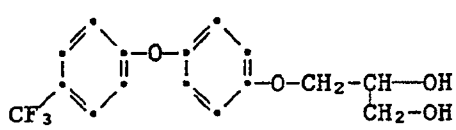

Smp. 87-88°C

b) Herstellung von 4-[4-(4-Fluorphenoxy)]-phenoxymethyl-2-äthyl-1,3-dioxolan

Zu einer Lösung von 13,9 g des nach a) hergestellten 1,2-Dihydroxy-3-[4-(4-fluorphenoxy)]-phenoxypropans und 40 mg p-Toluolsulfonsäure in 75 ml Cyclohexan werden unter Rühren bei Rückflusstemperatur innerhalb von 10 Minuten 8 g Propionaldehyd-diäthylacetal getropft. Nach einstündigem Rühren bei dieser Temperatur wird innerhalb ca. 2 1/2 Stunden das gebildete Aethanol fortlaufend über eine kurze Vigreuxkolonne abdestilliert. Dann wird das Reaktionsgemisch mit 10 %iger Natriumcarbonat-Lösung und anschliessend mit Wasser gewaschen, die Cyclohexan-Lösung über Natriumsulfat getrocknet, das Cyclohexan am Wasserstrahlvakuum abdestilliert und der Rückstand an Kieselgel chromatographiert (Eluierungsmittel: n-Hexan/Diäthyläther 19:1). Die auf diese Weise erhaltene Titelverbindung (Verbindung Nr. 1) der Formel

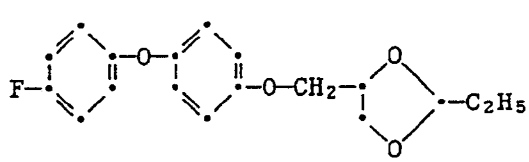

hat einen Schmelzpunkt von 47-48°C (Diasteromerengemisch).

Beispiel 2:

Herstellung von 4-[4-(4-Fluorphenoxy)]-phenoxymethyl-2-isopropyl1,3-dioxolan:

Eine Lösung von 13,9 g 1,2-Dihydroxy-3-[4-(4-fluorphenoxy)]-phenoxypropan, 5,1 g Isobutyraldehyd und 50 mg p-Toluolsulfonsäure in 150 ml Benzol werden während 5 Stunden unter Rühren am Wasserabscheider erhitzt. Anschliessend wird das Reaktionsgemisch wiederholt zunächst mit 10 %iger Natriumcarbonatlösung und dann mit Wasser gewaschen und über Natriumsulfat getrocknet und filtriert. Von dem erhaltenen Filtrat wird das Benzol im Vakuum abdestilliert. Das Rohprodukt wird an Kieselgel chromatographiert (Eluierungsmittel: Aether/n-Hexan 1:20), wobei die reine Titelverbindung vom Smp. 62-64°C (Verbindung Nr. 2) der Formel

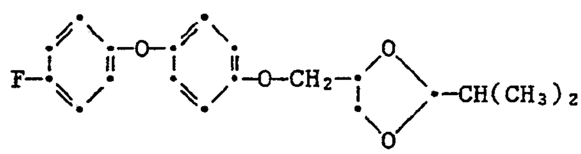

als Diastereomerengemisch erhalten wird.

Beispiel 3:

Herstellung von 4-[4-(4-Fluorphenoxy)]-phenoxymethyl-2,2-dimethyl-1,3-dioxolan:

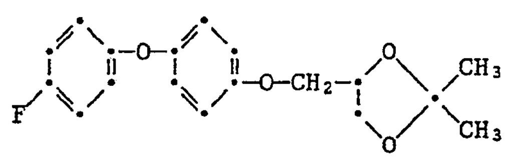

a) Es werden 6,4 g 4-(4-Fluorphenoxy)-phenol mit 3,5 g Kalium-tert.-butoxyd in wasserfreiem Dimethylsulfoxid umgesetzt, wodurch eine Lösung des Kaliumsalzes dieses Phenols erhalten wird. Zu dieser Lösung tropft man in einer Stickstoffatmosphäre bei 10°C unter Rühren die Lösung von 9,1 g R-(-)-2,2-Dimethyl-1,3-dioxolan-4-methanol-toluol-4-sulfonat in 20 ml Dimethylsulfoxid und rührt hierauf weitere 20 Stunden bei 20-22°C.

Zur Aufarbeitung wird das Reaktionsgemisch in 300 ml Eiswasser gegossen und viermal mit 100 ml Diäthyläther extrahiert. Die vereinigten Aetherphasen werden wiederholt mit Wasser gewaschen und über Natriumsulfat getrocknet. Nach dem Abdestillieren des Aethers wird der Rückstand an 250 g Kieselgel chromatographisch weiter gereinigt (Eluierungsmittel: Diäthylther-Hexan/1:20), wobei reines (S)-4-[4-(4-Fluorphenoxy)]-phenoxymethyl-2,2-dimethyl-1,3-dioxolan erhalten wird: $n_D^{20}$ = 1,5345, $[\alpha]_D^{20}$: + 5,2 ± 0,3° ($CHCl_3$) (Verbindung Nr. 3).

b) Analog wird aus dem Kaliumsalz des 4-(4-Fluorphenoxy)-phenols und S-(+)-2,2-Dimethyl-1,3-dioxolan-4-methanol-toluol-4-sulfonat in Dimethylsulfoxid das enantiomere (R)-4-[4-Fluorphenoxy)]-phenoxymethyl-2,2-dimethyl-1,3-dioxolan erhalten: $n_D^{20}$ = 1,5350, $[\alpha]_D^{20°}$: - 5,0 ± 0,3° ($CHCl_3$) (Verbindung Nr. 3a).

Die optische Reinheit der beiden unter a) und b) vorstehend charakterisierten Enantiomeren wurde durch MPLC-Chromatographie (medium pressure liquid chromatography) an Cellulosetriacetat überprüft. Sie beträgt ~ 99,8 % für die S-Verbindung und ~ 99,4 % für die R-Verbindung.

Analog der vorstehend beschriebenen Arbeitsweisen werden ferner die folgenden erfindungsgemässen Verbindungen der Formel Ia

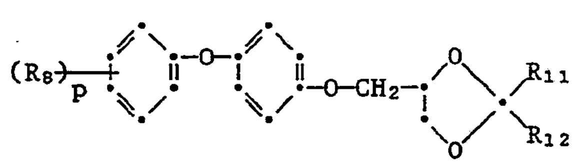

(Ia)

hergestellt, wobei sämtliche Verbindungen - ausser in den angegebenen Fällen - in Form ihrer Diastereomerengemische vorliegen:

| Verb. Nr. | $R_8$ | p | $R_{11}$ | $R_{12}$ | physik. Daten |
|---|---|---|---|---|---|
| 4 | 4-F | 1 | $-CH=CH_2$ | H | $n_D^{21}=1,5477$ |
| 5 | 4-F | 1 | $-C\equiv CH$ | H | Smp.74-76°C |
| 6 | 4-F | 1 | $-CH_3$ | $-CH_3$ | Smp.84-86°C *) |
| 7 | 3-F | 1 | $-CH=CH_2$ | H | $n_D^{20}=1,5493$ |
| 8 | 3-F | 1 | $-CH_3$ | $-CH_3$ | Smp.60-62°C *) |
| 9 | 3-F | 1 | $-C_2H_5$ | H | $n_D^{20}=1,5379$ |
| 10 | 2-F | 1 | $-C_2H_5$ | H | $n_D^{20}=1,5361$ |
| 11 | 4-F | 1 | $-(CH_2)_2-CH_3$ | H | $n_D^{20}=1,5301$ |
| 12 | 3-F | 1 | $-(CH_2)_2-CH_3$ | H | $n_D^{20}=1,5321$ |
| 13 | 4-F | 1 | $-CH_2-CH(CH_3)_2$ | H | $n_D^{20}=1,5257$ |
| 14 | 3-F | 1 | $-CH(CH_3)_2$ | H | Smp.ca.32°C |
| 15 | 2-F | 1 | $-(CH_2)_2-CH_3$ | H | $n_D^{20}=1,5318$ |
| 16 | 2-F | 1 | $-CH(CH_3)_2$ | H | $n_D^{20}=1,5321$ |
| 17 | 4-F | 1 | $-(CH_2)_5-$ | | Smp.53-55°C *) |
| 18 | 3-F | 1 | $-CH=CH-CH_3$ | H | $n_D^{20}=1,5443$ |
| 19 | 4-F | 1 | $-CH_2-\underset{OCH_3}{CH}-CH_3$ | H | $n_D^{20}=1,5281$ |
| 20 | 4-F | 1 | $-C_2H_5$ | $-CH_3$ | Smp.57-59°C |
| 21 | 2-F | 1 | $-CH_3$ | H | $n_D^{20}=1,5410$ |

*) kein Diastereomerengemisch, aber Racemat

| Verb. Nr. | $R_8$ | p | $R_{11}$ | $R_{12}$ | physik. Daten |
|---|---|---|---|---|---|
| 22 | 2-F | 1 | $-CH_3$ | H | $n_D^{23}$=1,5410 |
| 23 | 3-F | 1 | $-CH_2=CH-CH_3$ | H | $n_D^{20}$=1,5443 |
| 24 | 3-F | 1 | $-CH_2CH(OCH_3)-CH_3$ | H | $n_D^{20}$=1,5300 |
| 25 | 4-F | 1 | $-CH_3$ | H | Diastereomer A: Smp.46-47°C |
| 25a | 4-F | 1 | $-CH_3$ | H | Diastereomer B: $n_D^{21}$=1,5428 |
| 26 | 4-F | 1 | $-CH(CH_3)-C_2H_5$ | H | $n_D^{19}$=1,5369 |
| 27 | 2-F,4-F | 2 | $-C_2H_5$ | H | $n_D^{20}$=1,5280 |
| 28 | 2-F,4-F | 2 | $-CH(CH_3)_2$ | H | $n_D^{20}$=1,5225 |
| 29 | 4-F | 1 | 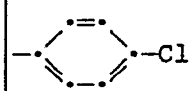 | H | Smp.89-91°C |
| 30 | 3-F | 1 | 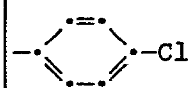 | H | Smp.59-61°C |
| 31 | 4-F | 1 | 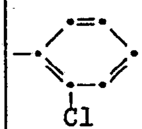 | H | $n_D^{23}$ =1,5782 |
| 32 | 4-F | 1 | 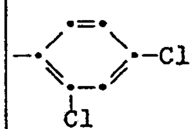 | H | $n_D^{20}$=1,5861 |
| 33 | 3-F | 1 | 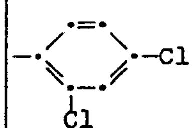 | H | $n_D^{21}$=1,5849 |
| 34 | 4-F | 1 | 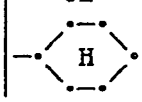 | $-CH_3$ | $n_D^{20}$=1,5359 |

| Verb. Nr. | $R_8$ | p | $R_{11}$ | $R_{12}$ | physik. Daten |
|---|---|---|---|---|---|
| 35 | 4-F | 1 | $-(CH_2)_5-$ | | Smp. 53-55°C*) |
| 36 | 4-F | 1 | 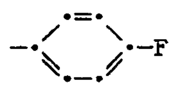 | H | Smp. 73-74°C |
| 37 | 4-F | 1 | $-C(CH_3)_3$ | H | $n_D^{22}$= 1,5379 |
| 38 | 4-F | 1 | $-(CH_2)_3-$ | | Smp. 72-73°C*) |
| 39 | 3-F | 1 | $-CH_2Cl$ | H | $n_D^{20}$=1,5504 |
| 40 | 3-F | 1 | $-(CH_2)_3-$ | | $n_D^{21}$=1,5414*) |
| 41 | 4-F | 1 | $-CH_2Br$ | H | $n_D^{20}$=1,5769 |
| 42 | 4-F | 1 | $-CO-OC_2H_5$ | H | $n_D^{20}$=1,5419 |
| 43 | 4-Cl | 1 | $-C_2H_5$ | H | $n_D^{20}$=1,5529 |
| 44 | 4-Cl | 1 | $-C_3H_7(n)$ | H | Smp.41-43°C |
| 45 | 4-F | 1 | $-CHCl_2$ | H | $n_D^{20}$=1,5669 |
| 46 | 3-F,5-F | 2 | $-CH=CH_2$ | H | Smp.46-48°C |
| 47 | 3-F,5-F | 2 | $-C_2H_5$ | H | Smp.32-34°C |
| 48 | 3-F,5-F | 2 | $-C_3H_7(n)$ | H | $n_D^{20}$=1,5262 |
| 49 | 3-F | 1 | $-C(CH_3)_3$ | H | $n_D^{20}$=1,5270 |
| 50 | 4-F | 1 | $-C(CH_3)_3$ | H | $n_D^{20}$=1,5289 |
| 51 | 3-F | 1 | $-CH_2-O-CH_3$ | H | $n_D^{20}$=1,5390 |
| 52 | 3-F | 1 | 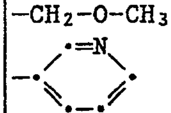 | H | $n_D^{20}$=1,5733 |
| 53 | 4-F | 1 | 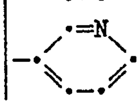 | H | Smp.60-63°C |

*) kein Diastereomerengemisch, aber Racemat

| Verb. Nr. | $R_8$ | p | $R_{11}$ | $R_{12}$ | physik. Daten |
|---|---|---|---|---|---|
| 54 | 3-F | 1 | $-NH-CO-OCH_3$ | H | Smp. 65-66°C |
| 55 | 3-F | 1 | $-CH=C(CH_3)_2$ | H | Smp. 67-69°C |
| 56 | 3-F | 1 | $-C\equiv CH$ | H | $n_D^{20}$=1,5539 |
| 57 | 3-F | 1 | $-(CH_2)_5-CH_3$ | H | $n_D^{20}$=1,5245 |
| 58 | 3-F | 1 | $-(CH_2)_4-CH_3$ | H | $n_D^{20}$=1,5260 |
| 59 | 3-F | 1 | $-C_4H_9(n)$ | H | $n_D^{22}$=1,5290 |
| 60 | 3-F | 1 | 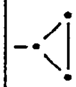 | $-CH_3$ | Diastereomer A (als Racemat): Smp. 69-70°C |
| 60a | 3-F | 1 | 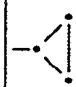 | $-CH_3$ | Diastereomer B (als Racemat): Smp. 44-46°C |
| 61 | 3-F | 1 | $-CH=CH_2$ | $-CH_3$ | Diastereomer A (als Racemat): $n_D^{22}$=1,5370 |
| 61a | 3-F | 1 | $-CH=CH_2$ | $-CH_3$ | Diastereomer B (als Racemat): $n_D^{22}$=1,5350 |
| 62 | 3-Cl | 1 | $-C_2H_5$ | H | $n_D^{20}$=1,5560 |
| 63 | 3-F | 1 | $-CH_2-C\equiv N$ | H | Smp. 85-87°C |
| 64 | 3-F, 5-F | 2 | $-CH(CH_3)_2$ | H | $n_D^{20}$=1,5221 |
| 65 | 2-F, 4-F | 2 | $-CH=CH_2$ | H | $n_D^{22}$=1,5376 |
| 66 | 3-Cl | 1 | $-C_3H_7(n)$ | H | $n_D^{22}$=1,5481 |
| 67 | 3-Cl | 1 | $-CH=CH_2$ | H | $n_D^{22}$=1,5639 |
| 68 | 2-F | 1 | 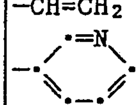 | H | Diastereomer A: $n_D^{20}$=1,5862 |
| 68a | | | | | Diastereomer B: $n_D^{20}$=1,5822 |
| 69 | 4-CF₃ | 1 | $-C_2H_5$ | H | Smp. 62-63°C |
| 70 | 3-F | 1 | $-CCl_2-CF_3$ | H | $n_D^{20}$=1,5695 |
| 71 | 3-F | 1 | $-CH_2-CH_2Cl$ | H | $n_D^{20}$=1,5341 |
| 72 | 3-F | 1 | $-(CH_2)_4-$ | | Smp. 94-95°C*) |

*) kein Diastereomerengemisch, aber Racemat

| Verb. Nr. | $R_8$ | p | $R_{11}$ | $R_{12}$ | physik. Daten |
|---|---|---|---|---|---|
| 73 | 3-F | 1 | 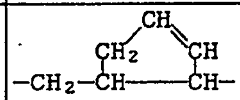 | | Smp. 36-38°C |
| 74 | 4-F | 1 | 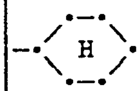 | H | $n_D^{22}$=1,5350 |
| 75 | 2-F | 1 | $-CH_2Cl$ | H | $n_D^{21}$=1,5618 |
| 76 | 4-F | 1 | $-(CH_2)_4-$ | | Smp.88-90°C*) |
| 77 | 4-F | 1 | $-CH_2Cl$ | H | $n_D^{22}$=1,5381 |
| 78 | 4-F | 1 | 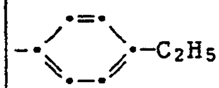 | H | Smp.84-85°C |
| 79 | 4-F | 1 | 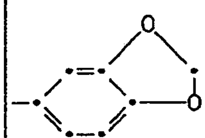 | H | Smp.83-85°C |
| 80 | 3-F | 1 | 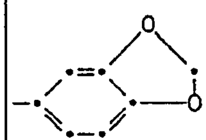 | H | Smp.47-50°C |
| 81 | 4-F | 1 | H | H | Smp.34-36°C*) |
| 82 | 3-F | 1 | $-CH_3$ | H | $n_D^{20}$=1,5420 |
| 83 | 3-F | 1 | $-C(CH_3)=CH_2$ | H | Smp.48-50°C |
| 84 | 3-F | 1 | $-(CH_2)_4-$ | | Smp.94-95°C*) |
| 85 | 3-F | 1 | 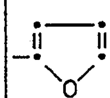 | H | $n_D^{22}$=1,5620 |
| 86 | 3-F | 1 | 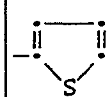 | H | Smp.51-53°C |
| 87 | 3-F | 1 | $-C_2H_5$ | H | Smp.84-86°C |
| 88 | 3-F | 1 | 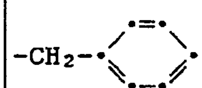 | H | Smp.~30°C |

*) kein Diastereomerengemisch, aber Racemat

Analog der vorstehend beschriebenen Arbeitsweisen werden auch die folgenden Verbindungen der Formel I hergestellt:

| Verbindung Nr. | | physik. Daten |
|---|---|---|
| 89 | 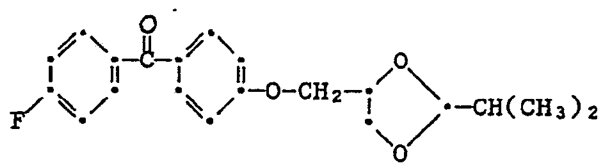 | Smp. 112-114°C |
| 90 | 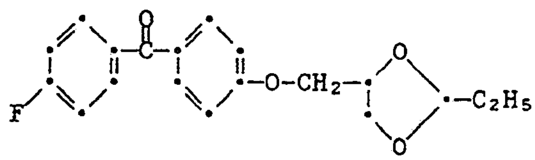 | Smp. 55-57°C |
| 91 | 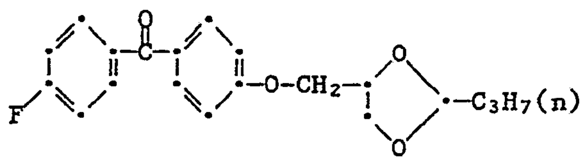 | Smp. 54-57°C |
| 92 | 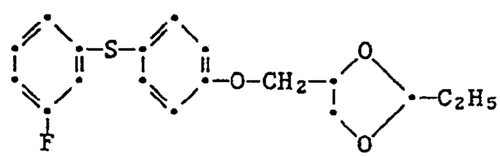 | $n_D^{20}$=1,5479 |
| 93 | 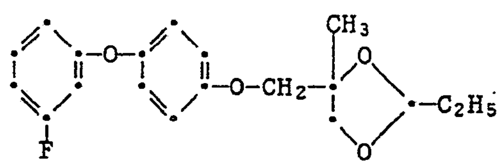 | $n_D^{20}$=1,5309 |
| 94 | 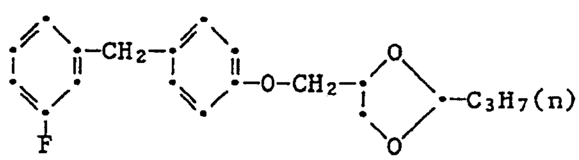 | Smp.56-57°C |

Auf analoge Weise wie vorstehend angegeben können die folgenden Verbindungen der Formel I hergestellt werden:

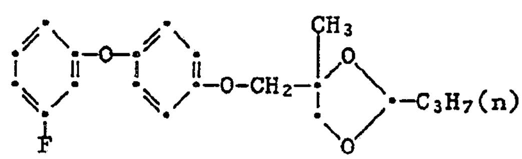

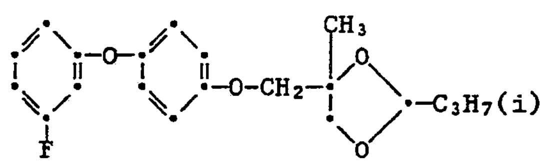

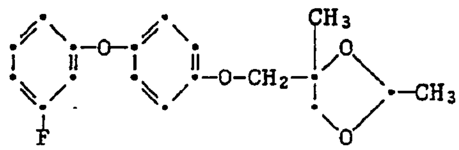

Beispiel 4:

Formulierungen für flüssige Wirkstoffe der Formel I gemäss den Beispielen 1 bis 3 (% = Gewichtsprozent)

| 1. Emulsions-Konzentrate | a) | b) | c) |
|---|---|---|---|
| Wirkstoff gemäss Herstellungsbeispielen | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff gemäss Herstellungsbeispielen | 80 % | 10 % | 5 % | 95 % |
| Aethylenglykol-monomethyläther | 20 % | - | - | - |
| Polyäthylenglykol (MG 400) | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 3. Granulate | a) | b) |
|---|---|---|
| Wirkstoff gemäss Herstellungsbeispielen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff gemäss Herstellungsbeispielen | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungen für feste Wirkstoffe der Formel I gemäss den Beispielen 1 bis 3 (% = Gewichtsprozent)

| 5. Spritzpulver | a) | b) | c) |
|---|---|---|---|
| Wirkstoff gemäss Herstellungsbeispielen | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | a) | b) |
|---|---|---|
| Wirkstoff gemäss Herstellungsbeispielen | 10 % | 10 % |
| Octylphenolpolyäthylenglykoläther (4-5 Mol AeO) | 3 % | - |
| Ca-Dodecylbenzolsulfonat | 3 % | - |
| Ricinusölpolyglykoläther (36 Mol AeO) | 4 % | - |
| Ricinusölthioxilat | - | 25 % |
| Cyclohexanon | 30 % | - |
| Butanol | - | 15 % |
| Xylolgemisch | 50 % | - |
| Essigester | - | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff gemäss Herstellungsbeispielen | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| 8. Extruder Granulat | |
|---|---|
| Wirkstoff gemäss Herstellungsbeispielen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 9. Umhüllungs-Granulat | |
|---|---|
| Wirkstoff gemäss Herstellungsbeispielen | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 10. Suspensions-Konzentrat | |
|---|---|
| Wirkstoff gemäss Herstellungsbeispielen | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

<u>Beispiel 5: Wirkung gegen Lucilia sericata</u>

Zu 9 ml eines Zuchtmediums wird bei 50°C ein ml einer 0,1 % Aktivsubstanz enthaltenden wässrigen Zubereitung hinzugefügt. Nun werden ca. 30 frisch geschlüpfte Lucilia sericata-Larven zum Zuchtmedium gegeben.

Nach 48 und 96 Stunden wird die insektizide Wirkung durch Ermittlung der Abtötungsrate festgestellt. Verbindungen der Formel I gemäss den Beispielen 1 bis 3 zeigen in diesem Test gute Wirkung gegen *Lucilia sericata*.

Beispiel 6: Wirkung gegen Aëdes aegypti

Auf die Oberfläche von 150 ml Wasser, das sich in einem Behälter befindet, wird so viel einer 0,1%igen acetonischen Lösung des Wirkstoffs pipettiert, dass eine Konzentration von 100 ppm erhalten wird. Nach Verdunsten des Acetons wird der Behälter mit 30 bis 40 2-tägigen Aëdes-Larven beschickt. Nach 2 und 7 Tagen wird die % Mortalität (Anzahl der nicht mehr schwimmfähigen Larven) geprüft. Verbindungen der Formel I gemäss den Beispielen 1 bis 3 zeigen gute Wirkung (Mortalität) im obigen Test.

Beispiel 7: Wirkung gegen Aonidiella aurantii

a) Tauchtest - Kartoffelknollen

Kleine Kartoffelknollen werden mit Wanderlarven ("crawlern") von Aonidiella aurantii (rote Citrus-Schildlaus) in der Weise infestiert, dass man die Kartoffeln während etwa 24 Stunden in direktem Kontakt mit stark von Aonodiella befallenen grossen Kürbissen hält, so dass schliesslich jede Kartoffel mit 200 bis 300 "crawlern" besiedelt wird. Wenn die Schildlaus Population auf den Kartoffeln das 2. Nymphenstadium erreicht hat, d.h. nach etwa 14 Tagen, werden die Kartoffeln für etwa 2 bis 3 Minuten mittels einer Zange in eine wässrige Emulsionszubereitung eingetaucht, die den Wirkstoff in einer Konzentration von 50 ppm enthält. Nach dem Trocknen der so behandelten Kartoffelknollen werden diese während 10 bis 12 Wochen in Plastikbehälter gebracht, die im oberen Teil mit einem Leimring versehen sind, welcher zum Abfangen der geflügelten Männchen dient. Danach wird zur Auswertung der Zustand der behandelten Schildlaus-Population mit demjenigen von unbehandelten Kontrollansätzen verglichen, wobei im einzelnen auf die Anzahl der Männchen, Entwicklung der Weibchen (Ausbildung von Schilden) und Produktion von "crawlern" der ersten Folgegeneration bonitiert wird.

b) Sprühbehandlung - Citruspflanzen

Stecklinge von Citrus trifoliata werden mit einem stark von Aonodiella befallenen Kürbis (vgl. vorstehend Test a) in engen Kontakt gebracht, so dass jeder Steckling mit etwa 150 bis 200 übergewanderten "crawlern" besiedelt wird. Wenn die Schildlaus-Population auf den Stecklingen das 2. Nymphenstadium erreicht hat, d.h. nach etwa 14 Tagen, werden die infestierten Stecklinge bis zur Tropfnässe mit einer wässrigen Emulsionszubereitung besprüht, die 50 ppm des zu prüfenden Wirkstoffes enthält. Nach etwa 10 bis 12 Wochen wird auf die Entwicklung der Population (%-Anteil männliche und weibliche Schilde) und Produktion von "crawlern" gegenüber unbehandelten Kontrollen bonitiert.

Verbindungen Nr. 1-19, 24, 26-34, 36-40, 74 und 76-82 der Formel I gemäss den Beispielen 1 bis 3 zeigen 100 % Wirkung in diesen Testen.

Beispiel 8: Insektizide Frassgift-Wirkung

Ca. 25 cm hohe eingetopfte Baumwollpflanzen werden mit einer wässrigen Wirkstoffemulsion besprüht, die den jeweiligen Wirkstoff in einer Konzentration von 400 ppm enthält.

Nach dem Antrocknen des Sprühbelages werden pro Konzentration je 3 Baumwollpflanzen in einem Metallkessel gestellt und mit 50 Spodoptera littoralis-Larven im ersten larvalen Stadium besiedelt. Der Kessel wird dann mit einer Glasplatte abgedeckt. Der Versuch wird bei 28°C und etwa 60 % relativer Luftfeuchtigkeit durchgeführt. Nach 96 Stunden wird die %-Mortalität der Test-Insekten gegenüber unbehandelten Kontrollen bestimmt.

Verbindungen der Formel I gemäss den Beispielen 1 bis 3 zeigen gute Wirkung in diesem Test.

Beispiel 9: Wirkung gegen Zecken

Als Testtiere werden mit Blut vollgesogene adulte Weichen der Rinderzecke Boophilus microplus verwendet. Es werden je 10 Zecken eines OP-resistenten Stammes (z.B. vom Biarra-Stamm) und eines normal empfindlichen Stammes (z.B. vom Yeerongpilly-Stamm) behandelt. Die Zecken werden auf mit Doppelklebeband bezogenen Platten dorsal fixiert und dann mit einem mit wässrigen Emulsionen bzw. Lösungen enthaltend 400 ppm der zu untersuchenden Verbindung getränkten Wattebausch während 1 Stunde bedeckt. Nach Entfernung des Wattebausches werden die Zecken über Nach bei 24°C getrocknet und anschliessend in einem klimatisierten Raum bei konstanten Bedingungen (28°C, 80 % rel. Luftfeuchtigkeit) gehalten, und zwar für 4 Wochen bis zum Ende der Eiablage und Beginn des Larvenschlupfes. Zur Auswertung wird die Mortalität, die prozentuale Hemmung der Ablage von fertilen Eiern (Blockierung der Embryogenese bzw. des Schlupfes) gegenüber unbehandelten Kontrollen ermittelt.

Die Verbindungen Nr. 1-10, 12-20, 24, 26, 27, 38, 76, 77 und 82 der Formel I gemäss Beispiel 1 bis 3 zeigen 100 % Wirkung in obigem Test.

Beispiel 10: Wirkung gegen Zecken: Abtötungswirkung in verschiedenen Entwicklungsstadien

Als Testobjekte werden Larven (jeweils ca. 50), Nymphen (jeweils ca. 25) oder Imagines (jeweils ca. 10) der Zeckenarten Rhipicephalus bursa, Amblyomma hebraeum und Boophilus microplus verwendet. Die Testtiere werden für kurze Zeit in wässrige Emulsionen der zu untersuchenden Substanzen mit einer Konzentration von 800 ppm getaucht. Die in Teströhrchen befindlichen Emulsionen werden dann mit Watte aufgenommen und die benetzten Testtiere in den so kontaminierten Röhrchen belassen. Die Auswertung (% - Mortalität) erfolgt für Larven nach 3 Tagen und für Nymphen und Imagines nach 14 Tagen.

Verbindungen der Formel I gemäss den Beispielen 1 bis 3 sind in diesem Test gut wirksam.

Beispiel 11: Ovizide Wirkung auf Cydia pomonella, Adoxophyes reticulana und Lobesia botrana

Abgelegte Eier der obigen Obstschädlinge, die nicht älter als 24 Stunden sind, werden dreimal für einige Sekunden in eine acetonischwässrige Lösung enthaltend 400 ppm des zu prüfenden Wirkstoffes eingetaucht. Nach dem Antrocknen der Test-Lösung werden die Eier in Petrischalen (5 cm Durchmesser) ausgelegt und bei einer Temperatur von etwa 26°C und 55 % relativer Luftfeuchtigkeit belassen. Im Falle von Cydia pomonella (Apfelwickler) werden die behandelten Eigelege zwischen zwei Papierrundfiltern in der Petrischale deponiert. Im Falle von Adoxophyes reticulana (Fruchtschalenwickler) und Lobesia botrana (Traubenwickler) werden die Eigelege zwischen zwei Stoffrundfiltern unter dem Deckel der Petrischale deponiert, deren Unterteil mit einer normalen Lepidopteren-Diät ausgegossen ist. Nach 6 Tagen wird der prozentuale Schlupf von Larven aus den behandelten Eiern gegenüber unbehandelten Kontrollpflanzen bewertet.

Verbindungen der Formel I gemäss Beispiel 1 bis 3 zeigen gute Wirkung in obigem Test.

Beispiel 12: Chemosterilisierungs-Wirkung auf Nilaparvata lugens

Der Test wird an wachsenden Pflanzen durchgeführt. Jeweils 4 Reispflanzen (Dicke des Stengels ca. 5 mm, Höhe ca. 20 cm) werden in Töpfe (Durchmesser von 8 cm) eingepflanzt.

Die Pflanzen werden auf einem Drehteller mit 5 ml einer wässrigen Emulsionszubereitung enthaltend 400 ppm des jeweiligen Wirkstoffes besprüht. Nach dem Antrocknen des Sprühbelages wird jede Pflanze mit 4 adulten Weibchen und 2 Männchen besiedelt. Um die Tiere am Entweichen zu hindern, wird über jeden besiedelten Topf ein durchsichtiger Kunststoff-Zylinder gestülpt und dieser mit einem Gaze-Deckel abgedeckt. Die Test-Tiere bleiben 6 Tage zur Eiablage auf den behandelten Pflanzen. Die überlebenden Tiere werden ausgezählt und dann entfernt.

Die Reispflanzen mit den abgelegten Eiern werden während 14 Tagen bei 26°C und 60 % relativer Luftfeuchtigkeit inkubiert. Die in diesem Zeitraum geschlüpften jungen Zikaden werden ausgezählt. Aus dem Vergleich der Anzahl geschlüpfter Larven auf den behandleten Pflanzen zu den auf unbehandelten Kontrollpflanzen geschlüpften Tieren wird die prozentuale Reduktion der Nachkommenschaft (Chemosterilisierungs-Effekt) bestimmt.

Verbindungen der Formel I gemäss den Beispielen 1 bis 3 zeigen in obigem Test gute Wirkung.

Beispiel 13: Wirkung gegen Bemisia tabaci

a) Wirkstoffapplikation vor Besiedlung

Eingetopfte Baumwollpflanzen im Keimblattstadium werden bis zur Tropfnässe mit einer wässrigen Emulsionszubereitung des zu prüfenden Wirkstoffes (Konzentration 400 ppm) besprüht. Nach dem Antrocknen des Sprühbelages werden auf jeder Pflanze 40 Adulte Bemisia tabaci (Weisse Fliege) in Plastikzylindern auf den Pflanzen gehalten. Zur Auswertung wird die %-Mortalität der angesetzten Adulten drei Tage nach Besiedlung bestimmt, wonach die überlebenden Adulten entfernt werden. Eine zweite Auswertung erfolgt 24 Tage nach Besiedlung auf %-Mortalität der Nymphen, Puppen und Adulten der ersten Folgegeneration. Der Versuch wird in einem klimatisierten Raum bei 25°C und einer relativen Luftfeuchtigkeit von etwa 50 bis 60 % durchgeführt.

b) Wirkstoffapplikation nach Besiedlung

Eingetopfte Baumwollpflanzen (unbehandelt) im Keimblattstadium werden wie unter a) angegeben mit Bemisia tabaci besiedelt (40 ungesexte Adulte pro Pflanze). Nach erfolgter Eiablage während drei Tagen werden alle vorhandenen Adulten entfernt. Zehn Tage nach der Besiedlung, d.h. zu einem Zeitpunkt, wo sich etw 2/3 der Nymphen im ersten Nymphenstadium und 1/3 im zweiten Stadium befinden, werden die besiedelten Pflanzen bis zur Tropfnässe mit einer wässrigen Emulsions-Zubereitung des zu prüfenden Wirkstoffes (Konzentration 400 ppm) besprüht. Die Auswertung erfolgt 24 Tage nach der Besiedlung auf %-Mortalität der Nymphen, Puppen und Adulten. Der Versuch wird in einem klimatisierten Raum bei 25°C und einer relativen Luftfeuchtigkeit von etwa 50 % bis 60 % durchgeführt.

Die Verbindungen der Formel I gemäss den vorliegenden Beispielen 1 bis 3 zeigen gute Wirkung in diesen Testen.

Beispiel 14: Wirkung gegen Vorratsschädlinge - Sitophilus orizae und Rhizopertha dominica

Es werden 100 g Weizenkörner in Plastikbecher von 100 ml Inhalt mit je 1 ml einer wässrigen Emulsionszubereitung des zu prüfenden Wirkstoffs gründlich vermischt, wobei die verwendete wässrige Emulsionszubereitung eine solche Wirkstoffkonzentration aufweist, dass sich, bezogen auf das Gewicht der Weizenkörner, eine End-Wirkstoffkonzentration von 100 ppm ergibt. Dann werden pro Becher (gefüllt mit 100 g behandelter Weizenkörner) 25 ungesexte Adulte von Sitophilus orizae (Reiskäfer) bzw. Rhizopertha dominica (Getreidekapuziner) angesetzt. Nach der Besiedlung werden die Ansätze bei 26 bis 28°C und 60 bis 65 % relativer Luftfeuchtigkeit im Dunkeln gehalten. Die Auswertung erfolgt eine Woche nach Besiedlung auf %-Mortalität der angesetzten Adulten und 8 Wochen nach Besiedlung auf %-Reduktion der ersten Folgegeneration.

Die Verbindungen der Formel I gemäss den vorliegenden Beispielen 1 bis 3 zeigen gute Wirkung in diesem Test.

## Patentansprüche

1. Verbindung der Formel I

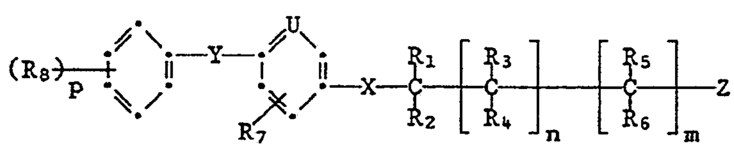

(I),

worin

$R_1$, $R_2$, $R_3$, $R_4$, $R_5$ und $R_6$ unabhängig voneinander Wasserstoff oder $C_1$-$C_4$-Alkyl oder, wenn n = 1,

$R_2$ und $R_4$ zusammen einen der Reste $-(CH_2)_3-$ oder $-(CH_2)_4-$ ;

$R_7$ Wasserstoff, Halogen, Methyl, Aethyl, mit 1 bis 5 Halogenatomen substituiertes $C_1$-$C_2$-Alkyl, Methoxy, Aethoxy oder mit 1 bis 5 Halogenatomen substituiertes $C_1$-$C_2$-Alkoxy;

$R_8$ Halogen, $C_1$-$C_3$-Alkyl, mit 1 bis 7 Halogenatomen substituiertes $C_1$-$C_3$-Alkyl, $C_1$-$C_3$-Alkoxy, mit 1 bis 7 Halogenatomen substituiertes $C_1$-$C_3$-Alkoxy oder Cyano;

U eine Gruppierung -CH= oder -N= ;

X -O- , -S- oder -N($R_9$)- ;

Y -O- , -S- , -S(O)- , -S($O_2$)- , -$CH_2$- , -CO- oder -N($R_9$)- , wobei

$R_9$ für Wasserstoff, $C_1$-$C_4$-Alkyl oder Alkylcarbonyl mit insgesamt 2 bis 4 C-Atomen steht;

n eine Zahl 0 oder 1;

m eine Zahl 0, 1 oder 2;

p eine Zahl 1, 2 oder 3; und

Z den Rest

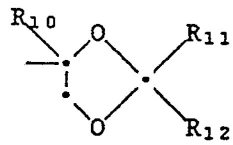

bedeuten, wobei

$R_{10}$ Wasserstoff, Methyl oder Aethyl;

$R_{11}$ Wasserstoff, $C_1$-$C_6$-Alkyl, $C_3$-$C_6$-Cycloalkyl, $C_2$-$C_4$-Alkenyl, $C_2$-$C_4$-Alkinyl, Alkoxyalkyl mit insgesamt 2 bis 6 C-Atomen, mit 1 bis 7 Halogenatomen substituiertes $C_1$-$C_6$-Alkyl, Benzyl, oder durch ein Halogenatom, einen $C_1$-$C_3$-Alkylrest oder $C_1$-$C_3$-Alkoxyrest kernsubstituiertes Benzyl, Cyanomethyl, ($C_1$-$C_2$-Alkoxy)-Carbonyl oder den Rest $-CH_2-NH-COO-(C_1$-$C_2$-Alkyl); und

$R_{12}$ Wasserstoff, $C_1$-$C_6$-Alkyl, Phenyl, mit 1 bis 3 Substituenten aus der Gruppe Halogen, $C_1$-$C_4$-Alkyl, mit 1 bis 7 Halogenatomen substituiertes $C_1$-$C_3$-Alkyl, $C_2$-$C_4$-Alkenyl, $C_2$-$C_4$-Alkinyl, $C_1$-$C_3$-Alkoxy, mit 1 bis 7 Halogenatomen substituiertes $C_1$-$C_3$-Alkoxy, Alkoxyalkyl mit insgesamt 2 bis 5 C-Atomen, $C_3$-$C_4$-Alkenyloxy, $C_3$-$C_4$-Alkinyloxy und $C_1$-$C_3$-Alkylthio substituiertes Phenyl, Pyridyl, Furan-2-yl, Thien-2-yl oder den Rest

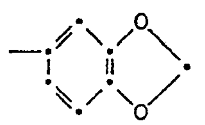 ;

oder

$R_{12}$ und $R_{11}$ zusammen eine der Gruppen $-(CH_2)_3-$, $-(CH_2)_4-$ oder $-(CH_2)_5-$ bedeuten.

2. Verbindung der Formel I gemäss Anspruch 1, dadurch gekennzeichnet, dass

$R_1$, $R_2$, $R_3$, $R_4$, $R_5$ und $R_6$ unabhängig voneinander Wasserstoff oder $C_1$-$C_4$-Alkyl oder, wenn n = 1,

$R_2$ und $R_4$ zusammen einen der Reste $-(CH_2)_3-$ oder $-(CH_2)_4-$ ;

$R_7$ Wasserstoff, Halogen, Methyl, Aethyl, mit 1 bis 5 Halogenatomen substituiertes $C_1$-$C_2$-Alkyl, Methoxy, Aethoxy oder mit 1 bis 5 Halogenatomen substituiertes $C_1$-$C_2$-Alkoxy;

$R_8$ Halogen, $C_1$-$C_3$-Alkyl, mit 1 bis 7 Halogenatomen substituiertes $C_1$-$C_3$-Alkyl, $C_1$-$C_3$-Alkoxy, mit 1 bis 7 Halogenatomen substituiertes $C_1$-$C_3$-Alkoxy oder Cyano;

U eine Gruppierung -CH= oder -N= ;

X -O- , -S- oder $-N(R_9)-$ ;

Y -O- , -S- , -S(O)- , $-S(O_2)-$ , $-CH_2-$ , -CO- oder $-N(R_9)-$ , wobei

$R_9$ für Wasserstoff, $C_1$-$C_4$-Alkyl oder Alkylcarbonyl mit insgesamt 2 bis 4 C-Atomen steht;

n eine Zahl 0 oder 1;

m eine Zahl 0, 1 oder 2;

p eine Zahl 1, 2 oder 3; und

Z den Rest

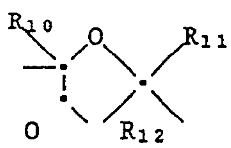

bedeuten, wobei

$R_{10}$ Wasserstoff, Methyl oder Aethyl;

$R_{11}$ Wasserstoff, $C_1$-$C_6$-Alkyl, $C_3$-$C_6$-Cycloalkyl, $C_2$-$C_4$-Alkenyl, $C_2$-$C_4$-Alkinyl, Alkoxyalkyl mit insgesamt 2 bis 6 C-Atomen, mit 1 bis 7 Halogenatomen substituiertes $C_1$-$C_6$-Alkyl, Benzyl, oder durch ein Halogenatom, einen $C_1$-$C_3$-Alkylrest oder $C_1$-$C_3$-Alkoxyrest kernsubstituiertes Benzyl;

$R_{12}$ Wasserstoff, $C_1$-$C_6$-Alkyl, Phenyl, mit 1 bis 3 Substituenten aus der Gruppe Halogen, $C_1$-$C_4$-Alkyl, mit 1 bis 7 Halogenatomen substituiertes $C_1$-$C_3$-Alkyl, $C_2$-$C_4$-Alkenyl, $C_2$-$C_4$-Alkinyl, $C_1$-$C_3$-Alkoxy, mit 1 bis 7

Halogenatomen substituiertes $C_1$-$C_3$-Alkoxy, Alkoxyalkyl mit insgesamt 2 bis 5 C-Atomen, $C_3$-$C_4$-Alkenyloxy, $C_3$-$C_4$-Alkinyloxy und $C_1$-$C_3$-Alkylthio substituiertes Phenyl oder den Rest

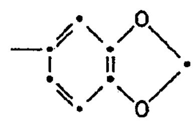 ;

oder

$R_{12}$ und $R_{11}$ zusammen einen der Reste $-(CH_2)_4-$ oder $-(CH_2)_5-$ bedeuten.

3. Verbindung der Formel I gemäss Anspruch 1 oder 2, dadurch gekennzeichnet, dass
$R_1$ und $R_3$ unabhängig voneinander Wasserstoff, Methyl oder Aethyl;
$R_2$, $R_4$, $R_5$ und $R_6$ Wasserstoff oder, wenn n = 1,
$R_2$ und $R_4$ zusammen einen der Reste $-(CH_2)_3-$ oder $-(CH_2)_4-$ ;
$R_7$ Wasserstoff oder Halogen;
$R_8$ Methyl, Aethyl, Halogen oder mit 1 bis 5 Halogenatomen substituiertes $C_1$-$C_2$-Alkyl;
U eine Gruppierung -CH= oder -N= ;
X -O- , -S- oder -N($R_9$)- ;
Y -O- , -S- , -$CH_2$- oder -CO- ; wobei
$R_9$ für Wasserstoff, Methyl oder Acetyl steht;
n und m unabhängig voneinander eine Zahl 0 oder 1; und
p eine Zahl 1 oder 2; und
Z den Rest

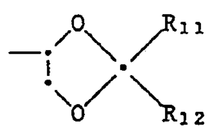

bedeuten, wobei
$R_{11}$ Wasserstoff, $C_1$-$C_4$-Alkyl, $C_3$-$C_6$-Cycloalkyl, $C_2$-$C_3$-Alkenyl, $C_2$-$C_3$-Alkinyl, Alkoxyalkyl mit insgesamt 2 bis 4 C-Atomen, mit 1 bis 7 Halogenatomen substituiertes $C_1$-$C_4$-Alkyl oder Benzyl; und
$R_{12}$ Wasserstoff, $C_1$-$C_4$-Alkyl, Phenyl, mit 1 oder 2 Substituenten aus der Gruppe Halogen, $C_1$-$C_3$-Alkyl, mit 1 bis 5 Halogenatomen substituiertes $C_1$-$C_2$-Alkyl, $C_2$-$C_4$-Alkenyl, $C_2$-$C_4$-Alkinyl, $C_1$-$C_3$-Alkoxy, mit 1 bis 3 Halogenatomen substituiertes Methoxy, Alkoxyalkyl mit insgesamt 3 bis 4 C-Atomen, $C_3$-$C_4$-Alkenyloxy, $C_3$-$C_4$-Alkinyloxy und $C_1$-$C_3$-Alkylthio substituiertes Phenyl oder den Rest

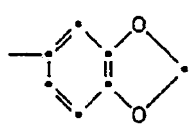 ;

oder

$R_{12}$ und $R_{11}$ zusammen einen der Reste $-(CH_2)_4-$ oder $-(CH_2)_5-$ bedeuten.

4. Verbindung der Formel I gemäss Anspruch 3, dadurch gekennzeichnet, dass
$R_1$ Wasserstoff oder Methyl;
$R_2$, $R_3$, $R_4$, $R_5$, $R_6$ und $R_7$ Wasserstoff;
$R_8$ Fluor, Chlor, Methyl oder Trifluormethyl;
U die Gruppierung -CH= ;
X -O- ;
Y -O- ;

n und m die Zahl 0;
p eine Zahl 1 oder 2; und
Z den Rest

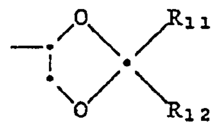

bedeuten, wobei
$R_{11}$ Wasserstoff, $C_1$-$C_4$-Alkyl, $C_3$-$C_6$-Cycloalkyl, $C_2$-$C_3$-Alkenyl, $C_2$-$C_3$-Alkinyl, Alkoxyalkyl mit insgesamt 2 bis 4 C-Atomen oder Benzyl; und
$R_{12}$ Wasserstoff, $C_1$-$C_4$-Alkyl, mit 1 bis 5 Halogenatomen substituiertes $C_1$-$C_2$-Alkyl, Phenyl, mit 1 oder 2 Substituenten aus der Gruppe Halogen und $C_1$-$C_3$-Alkyl, substituiertes Phenyl oder den Rest

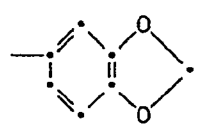 ;

oder

$R_{12}$ und $R_{11}$ zusammen einen der Reste $-(CH_2)_4-$ oder $-(CH_2)_5-$ bedeuten.

5. Verbindung der Formel I gemäss Anspruch 1, dadurch gekennzeichnet, dass
$R_1$ Wasserstoff oder Methyl;
$R_2$, $R_3$, $R_4$, $R_5$, $R_6$ und $R_7$ Wasserstoff
$R_8$ Halogen oder Trifluormethyl;
U die Gruppierung -CH=;
X -O- oder -S-;
Y -O- , -S- , $-CH_2-$ oder -CO-;
n und m die Zahl 0;
p eine Zahl 1 oder 2; und
Z den Rest

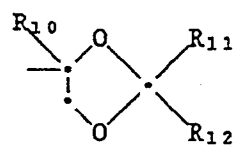

bedeuten, wobei
$R_{10}$ Wasserstoff oder Methyl;
$R_{11}$ Wasserstoff, $C_1$-$C_6$-Alkyl, $C_3$-$C_6$-Cycloalkyl, $C_2$-$C_4$-Alkenyl, $C_2$-$C_4$-Alkinyl, Alkoxyalkyl mit insgesamt 2 bis 4 C-Atomen, mit 1 bis 5 Halogenatomen substituiertes $C_1$-$C_4$-Alkyl, Benzyl, Cyanomethyl, ($C_1$-$C_2$-Alkoxy)-carbonyl oder den Rest $-CH_2$-NH-COO-($C_1$-$C_2$-Alkyl);und
$R_{12}$ Wasserstoff, $C_1$-$C_6$-Alkyl, mit 1 bis 2 Substituenten aus der Gruppe Halogen, $C_1$-$C_2$-Alkyl, $C_2$-$C_4$-Alkenyl, $C_2$-$C_3$-Alkinyl, $C_1$-$C_2$-Alkoxy, und $C_2$-$C_3$-Alkylthio substituiertes Phenyl, Pyridyl, Furan-2-yl, Thien-2-yl oder den Rest

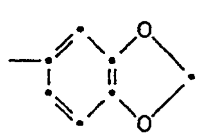 ;

oder

$R_{12}$ und $R_{11}$ zusammen eine der Gruppen $-(CH_2)_3-$, $-(CH_2)_4-$ oder $-(CH_2)_5-$ bedeuten.

6. Verbindung der Formel I gemäss Anspruch 5, dadurch gekennzeichnet, dass
$R_1$, $R_2$, $R_3$, $R_4$, $R_5$, $R_6$ und $R_7$ Wasserstoff;
$R_8$ Fluor, Chlor oder Trifluormethyl;
U die Gruppierung -CH=;
X -O-,
Y -O-, -S-, $-CH_2-$ oder -CO-;
n und m die Zahl 0;
p eine Zahl 1 oder 2; und
Z den Rest

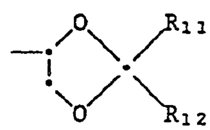

bedeuten, wobei
$R_{11}$ Wasserstoff, $C_1$-$C_6$-Alkyl, $C_3$-$C_6$-Cycloalkyl, $C_2$-$C_4$-Alkenyl, $C_2$-$C_4$-Alkinyl, Alkoxyalkyl mit insgesamt 2 bis 4 C-Atomen, mit 1 bis 5 Fluor- oder Chlor-atomen substituiertes $C_1$-$C_4$-Alkyl, Benzyl, Cyanomethyl, Aethoxycarbonyl oder den Rest $-CH_2-NH-COO-CH_3$; und
$R_{12}$ Wasserstoff, $C_1$-$C_6$-Alkyl, mit 1 bis 2 Substituenten aus der Gruppe Fluor, Chlor, $C_1$-$C_2$-Alkyl, und $C_1$-$C_2$-Alkoxy, substituiertes Phenyl, Pyrid-4-yl, Furan-2-yl, oder Thien-2-yl oder den Rest

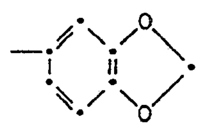 ;

oder

$R_{12}$ und $R_{11}$ zusammen eine der Gruppen $-(CH_2)_3-$, $-(CH_2)_4-$ oder $-(CH_2)_5-$ bedeuten.

7. Verbindung gemäss Anspruch 4 der Formel Ia

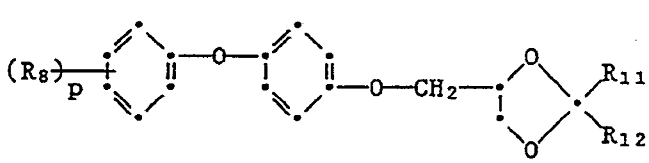 (Ia),

worin
$R_8$ Fluor, Chlor oder Trifluormethyl;
p eine Zahl 1 oder 2; und
$R_{11}$ und $R_{12}$ unabhängig voneinander Wasserstoff, $C_1$-$C_4$-Alkyl, $C_2$-$C_4$-Methoxyalkyl, mit 1 bis 3 Halogenatomen substituiertes $C_1$-$C_2$-Alkyl, $C_2$-$C_3$-Alkenyl oder $C_2$-$C_3$-Alkinyl oder $R_{11}$ und $R_{12}$ zusammen einen der Reste $-(CH_2)_4-$ oder $-(CH_2)_5-$ bedeuten.

8. Verbindung gemäss einem der Ansprüche 1 bis 7, dadurch gekennzeichnet, dass p = 1 ist.

9. Verbindung gemäss einem der Ansprüche 1 bis 8, dadurch gekennzeichnet, dass $R_{12}$ Wasserstoff bedeutet.

10. Verbindung gemäss Anspruch 9 der Formel

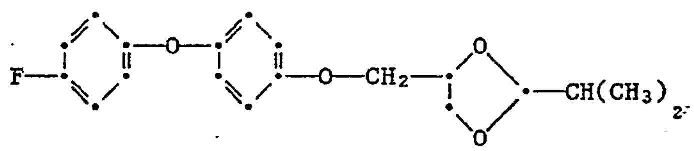

11. Verbindung gemäss Anspruch 8 der Formel

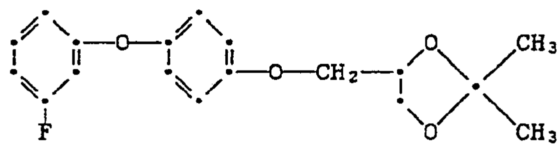

12. Verbindung gemäss Anspruch 9 der Formel

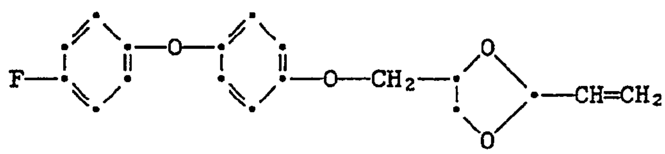

13. Verbindung gemäss Anspruch 9 der Formel

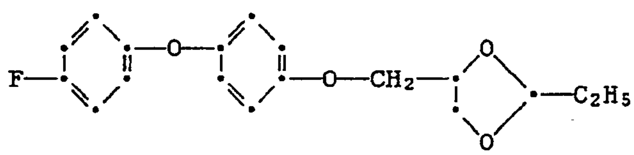

14. Verbindung gemäss Anspruch 9 der Formel

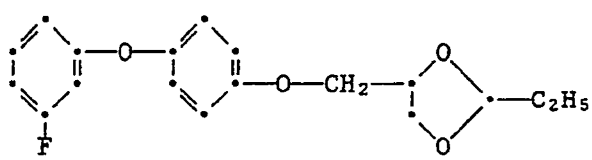

15. Verbindung gemäss Anspruch 9 der Formel

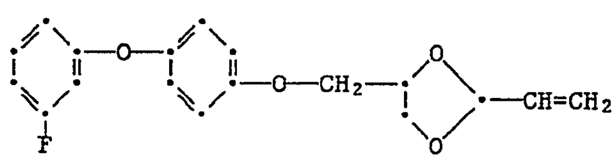

16. Verbindung gemäss Anspruch 9 der Formel

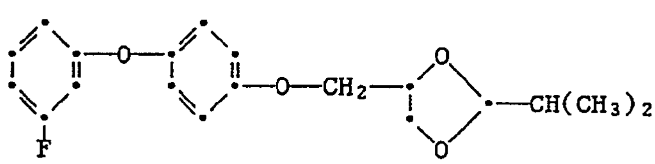

17. Verbindung gemäss Anspruch 8 der Formel

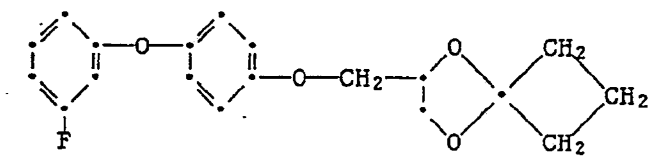

18. Verbindung gemäss Anspruch 9 der Formel

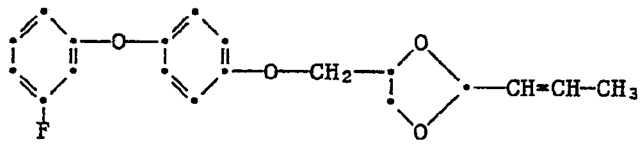

19. Verbindung gemäss Anspruch 7 der Formel

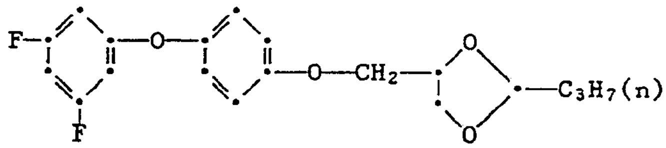

20. Verbindung gemäss Anspruch 9 der Formel

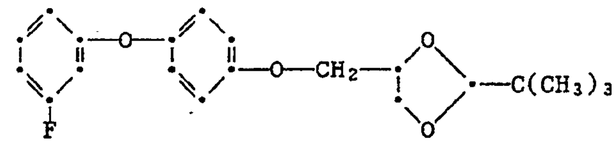 .

21. Verbindung gemäss Anspruch 7 der Formel

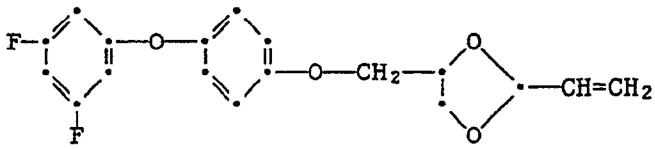 .

22. Verbindung gemäss Anspruch 5 der Formel

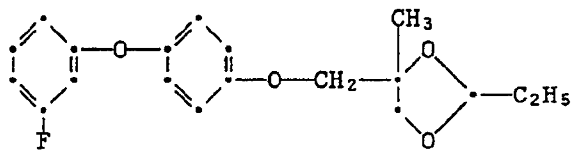 .

23. Verbindung gemäss Anspruch 9 der Formel

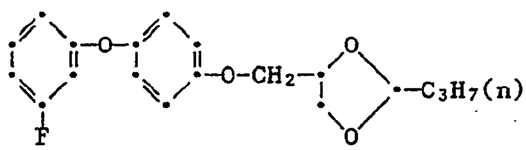 .

24. Verfahren zur Herstellung einer Verbindung der Formel I gemäss einem der Ansprüche 1 bis 23, dadurch gekennzeichnet, dass man eine Verbindung der Formel II

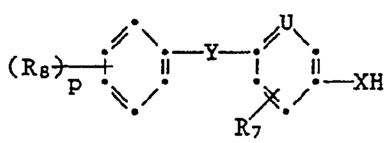 (II)

mit einer Verbindung der Formel III

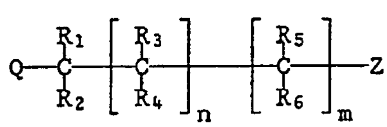 (III)

umsetzt, wobei in den Formeln II und III $R_1$ bis $R_8$, U, X, Y, n, m, p und Z die in Ansprüchen 1 bis 9 angegebenen Bedeutungen haben und Q für eine Abgangsgruppe steht.

25. Verfahren zur Herstellung einer Verbindung der Formel I gemäss einem der Ansprüche 1 bis 23, worin Z den Rest

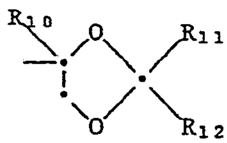

bedeutet, dadurch gekennzeichnet, dass man eine Verbindung der Formel IV

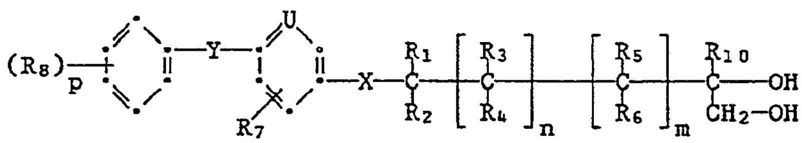 (IV)

in Gegenwart eines sauren Katalysators mit einer Verbindung der Formel V oder Va

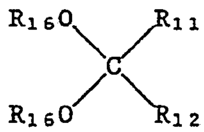 (V) 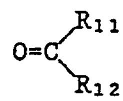 (Va)

kondensiert, wobei in den Formeln IV, V und Va $R_1$ bis $R_{10}$, U, X, Y, n, m, p, $R_{11}$ und $R_{12}$ die in den Ansprüchen 1 bis 9 angegebenen Bedeutungen haben und $R_{16}$ für Methyl oder Aethyl steht.

26. Verbindung der Formel IV gemäss Anspruch 25.

27. Schädlingsbekämpfungsmittel, welches als aktive Komponente eine Verbindung gemäss einem der Ansprüche 1 bis 23 zusammen mit geeigneten Trägern und/oder anderen Zuschlagstoffen enthält.

28. Verwendung einer Verbindung gemäss einem der Ansprüche 1 bis 23 zur Bekämpfung von Insekten und Vertretern der Ordnung Akarina.

29. Verwendung gemäss Anspruch 28 zur Bekämpfung von pflanzenschädigenden saugenden Insekten.

30. Verwendung gemäss Anspruch 29 zur Bekämpfung von Schildläusen.

31. Verwendung gemäss Anspruch 28 zur Bekämpfung larvaler Stadien pflanzenschädigender Insekten.

32. Verfahren zur Bekämpfung von Insekten und Vertretern der Ordnung Akarina, dadurch gekennzeichnet, dass man die Schädlinge, bzw. deren verschiedene Entwicklungsstadien oder deren Aufenthaltsort, mit einer pestizid wirksamen Menge einer Verbindung der Formel I gemäss einem der Ansprüche 1 bis 23 oder mit einem Mittel, enthaltend neben Zusatz- und Trägerstoffen eine pestizid wirksame Menge dieser Verbindung, in Kontakt bringt oder behandelt.

## Claims

1. A compound of formula I

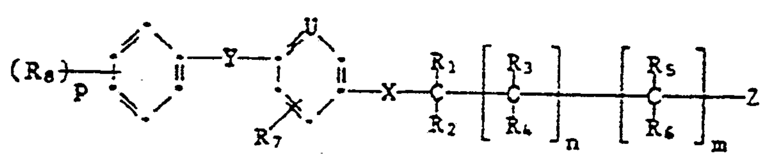 (I)

in which

$R_1$, $R_2$, $R_3$, $R_4$, $R_5$ and $R_6$ each independently of the others represents hydrogen or $C_1$-$C_4$-alkyl or, if n = 1, $R_2$ and $R_4$ together represent one of the radicals $-(CH_2)_3-$ or $-(CH_2)_4-$;

$R_7$ represents hydrogen, halogen, methyl, ethyl, $C_1$-$C_2$-alkyl substituted by 1 to 5 halogen atoms, methoxy, ethoxy or $C_1$-$C_2$-alkoxy substituted by 1 to 5 halogen atoms;

$R_8$ represents halogen, $C_1$-$C_3$-alkyl, $C_1$-$C_3$-alkyl substituted by 1 to 7 halogen atoms, $C_1$-$C_3$-alkoxy, $C_1$-$C_3$-alkoxy substituted by 1 to 7 halogen atoms, or cyano;

U represents a grouping -CH= or -N=;
X represents -O-, -S- or $-N(R_9)-$;
Y represents -O-, -S-, -S(O)-, $-S(O_2)-$, $-CH_2-$, -CO- or $-N(R_9)-$, wherein
$R_9$ represents hydrogen, $C_1$-$C_4$-alkyl or alkylcarbonyl having a total of 2 to 4 carbon atoms;
n is a number 0 or 1;
m is a number 0, 1 or 2;
p is a number 1, 2 or 3; and
Z represents the radical

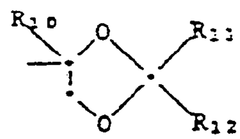

wherein
$R_{10}$ represents hydrogen, methyl or ethyl;
$R_{11}$ represents hydrogen, $C_1$-$C_6$-alkyl, $C_3$-$C_6$-cycloalkyl, $C_2$-$C_4$-alkenyl, $C_2$-$C_4$-alkynyl, alkoxyalkyl having a total of 2 to 6 carbon atoms, $C_1$-$C_6$-alkyl substituted by 1 to 7 halogen atoms, benzyl or benzyl substituted in the nucleus by a halogen atom, by a $C_1$-$C_3$-alkyl radical or by a $C_1$-$C_3$-alkoxy radical, or represents cyanomethyl, ($C_1$-$C_2$-alkoxy)-carbonyl or the radical $-CH_2-NH-COO-(C_1-C_2\text{-alkyl})$; and
$R_{12}$ represents hydrogen, $C_1$-$C_6$-alkyl, phenyl, phenyl substituted by 1 to 3 substituents from the group consisting of halogen, $C_1$-$C_4$-alkyl, $C_1$-$C_3$-alkyl substituted by 1 to 7 halogen atoms, $C_2$-$C_4$-alkenyl, $C_2$-$C_4$-alkynyl, $C_1$-$C_3$-alkoxy, $C_1$-$C_3$-alkoxy substituted by 1 to 7 halogen atoms, alkoxyalkyl having a total of 2 to 5 carbon atoms, $C_3$-$C_4$-alkenyloxy, $C_3$-$C_4$-alkynyloxy and $C_1$-$C_3$-alkylthio, or represents pyridyl, furan-2-yl, thien-2-yl or the radical

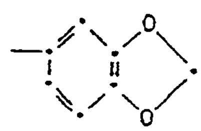 ;

or

$R_{12}$ and $R_{11}$ together represent one of the groups $-(CH_2)_3-$, $-(CH_2)_4-$ or $-(CH_2)_5-$.

2. A compound of formula I according to claim 1, characterised in that
$R_1$, $R_2$, $R_3$, $R_4$, $R_5$ and $R_6$ each independently of the others represents hydrogen or $C_1$-$C_4$-alkyl or, if n = 1, $R_2$ and $R_4$ together represent one of the radicals $-(CH_2)_3-$ or $-(CH_2)_4-$;
$R_7$ represents hydrogen, halogen, methyl, ethyl, $C_1$-$C_2$-alkyl substituted by 1 to 5 halogen atoms, methoxy, ethoxy or $C_1$-$C_2$-alkoxy substituted by 1 to 5 halogen atoms;
$R_8$ represents halogen, $C_1$-$C_3$-alkyl, $C_1$-$C_3$-alkyl substituted by 1 to 7 halogen atoms, $C_1$-$C_3$-alkoxy, $C_1$-$C_3$-alkoxy substituted by 1 to 7 halogen atoms, or cyano;
U represents a grouping -CH= or -N=;
X represents -O-, -S- or $-N(R_9)-$;
Y represents -O-, -S-, -S(O)-, $-S(O_2)-$, $-CH_2-$, -CO- or $-N(R_9)-$, wherein
$R_9$ represents hydrogen, $C_1$-$C_4$-alkyl or alkylcarbonyl having a total of 2 to 4 carbon atoms;
n is a number 0 or 1;
m is a number 0, 1 or 2;
p is a number 1, 2 or 3; and
Z represents the radical

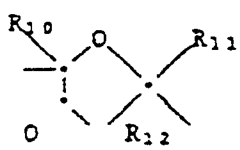

wherein
$R_{10}$ represents hydrogen, methyl or ethyl;
$R_{11}$ represents hydrogen, $C_1$-$C_6$-alkyl, $C_3$-$C_6$-cycloalkyl, $C_2$-$C_4$-alkenyl, $C_2$-$C_4$-alkynyl, alkoxyalkyl having a total of 2 to 6 carbon atoms, $C_1$-$C_6$-alkyl substituted by 1 to 7 halogen atoms, benzyl, or benzyl substituted in the nucleus by a halogen atom, by a $C_1$-$C_3$-alkyl radical or by a $C_1$-$C_3$-alkoxy radical;
$R_{12}$ represents hydrogen, $C_1$-$C_6$-alkyl, phenyl, phenyl substituted by 1 to 3 substituents from the group consisting of halogen, $C_1$-$C_4$-alkyl, $C_1$-$C_3$-alkyl substituted by 1 to 7 halogen atoms, $C_2$-$C_4$-alkenyl, $C_2$-$C_4$-alkynyl, $C_1$-$C_3$-alkoxy, $C_1$-$C_3$-alkoxy substituted by 1 to 7 halogen atoms, alkoxyalkyl having a total of 2 to 5 carbon atoms, $C_3$-$C_4$-alkenyloxy, $C_3$-$C_4$-alkynyloxy and $C_1$-$C_3$-alkylthio, or represents the radical

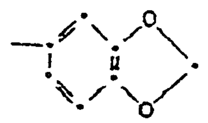
;

or

$R_{12}$ and $R_{11}$ together represent one of the radicals $-(CH_2)_4-$ or $-(CH_2)_5-$.
3. A compound of formula I according to claim 1 or 2, characterised in that
$R_1$ and $R_3$ each independently of the other represents hydrogen, methyl or ethyl;
$R_2$, $R_4$, $R_5$ and $R_6$ represent hydrogen or, if n = 1,

$R_2$ and $R_4$ together represent one of the radicals $-(CH_2)_3-$ or $-(CH_2)_4-$;
$R_7$ represents hydrogen or halogen;
$R_8$ represents methyl, ethyl, halogen or $C_1$-$C_2$-alkyl substituted by 1 to 5 halogen atoms;
U represents a grouping -CH= or -N=;
X represents -O-, -S- or -N($R_9$)-;
Y represents -O-, -S-, -$CH_2$- or -CO-; wherein
$R_9$ represents hydrogen, methyl or acetyl;
n and m each independently of the other is a number 0 or 1; and
p is a number 1 or 2; and
Z represents the radical

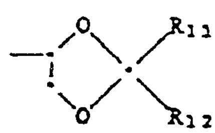

wherein
$R_{11}$ represents hydrogen, $C_1$-$C_4$-alkyl, $C_3$-$C_6$-cycloalkyl, $C_2$-$C_3$-alkenyl, $C_2$-$C_3$-alkynyl, alkoxyalkyl having a total of 2 to 4 carbon atoms, $C_1$-$C_4$-alkyl substituted by 1 to 7 halogen atoms, or benzyl; and
$R_{12}$ represents hydrogen, $C_1$-$C_4$-alkyl, phenyl, phenyl substituted by 1 or 2 substituents from the group consisting of halogen, $C_1$-$C_3$-alkyl, $C_1$-$C_2$-alkyl substituted by 1 to 5 halogen atoms, $C_2$-$C_4$-alkenyl, $C_2$-$C_4$-alkynyl, $C_1$-$C_3$-alkoxy, methoxy substituted by 1 to 3 halogen atoms, alkoxyalkyl having a total of 3 or 4 carbon atoms, $C_3$-$C_4$-alkenyloxy, $C_3$-$C_4$-alkynyloxy and $C_1$-$C_3$-alkylthio, or represents the radical

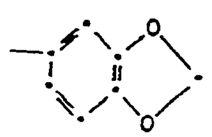
;

or

$R_{12}$ and $R_{11}$ together represent one of the radicals $-(CH_2)_4-$ or $-(CH_2)_5-$.
4. A compound of formula I according to claim 3, characterised in that

$R_1$ represents hydrogen or methyl;
$R_2$, $R_3$, $R_4$, $R_5$, $R_6$ and $R_7$ represent hydrogen;
$R_8$ represents fluorine, chlorine, methyl or trifluoromethyl;
U represents the grouping -CH=;
X represents -O-;
Y represents -O-;
n and m are the number 0;
p is a number 1 or 2; and
Z represents the radical

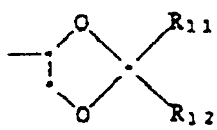

wherein
$R_{11}$ represents hydrogen, $C_1$-$C_4$-alkyl, $C_3$-$C_6$-cycloalkyl, $C_2$-$C_3$-alkenyl, $C_2$-$C_3$-alkynyl, alkoxyalkyl having a total of 2 to 4 carbon atoms, or benzyl; and
$R_{12}$ represents hydrogen, $C_1$-$C_4$-alkyl, $C_1$-$C_2$-alkyl substituted by 1 to 5 halogen atoms, phenyl, phenyl substituted by 1 or 2 substituents from the group consisting of halogen and $C_1$-$C_3$-alkyl, or represents the radical

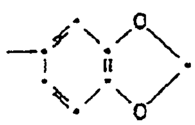 ;

or

$R_{12}$ and $R_{11}$ together represent one of the radicals $-(CH_2)_4-$ or $-(CH_2)_5-$.

5. A compound of formula I according to claim 1, characterised in that
$R_1$ represents hydrogen or methyl;
$R_2$, $R_3$, $R_4$, $R_5$, $R_6$ and $R_7$ represent hydrogen;
$R_8$ represents halogen or trifluoromethyl;
U represents the grouping -CH=;
X represents -O- or -S-;
Y represents -O-, -S-, $-CH_2-$ or -CO-;
n and m are the number 0;
p is a number 1 or 2; and
Z represents the radical

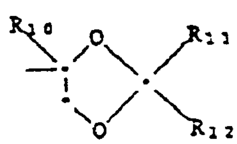

wherein
$R_{10}$ represents hydrogen or methyl;
$R_{11}$ represents hydrogen, $C_1$-$C_6$-alkyl, $C_3$-$C_6$-cycloalkyl, $C_2$-$C_4$-alkenyl, $C_2$-$C_4$-alkynyl, alkoxyalkyl having a total of 2 to 4 carbon atoms, $C_1$-$C_4$-alkyl substituted by 1 to 5 halogen atoms, benzyl, cyanomethyl, ($C_1$-$C_2$-alkoxy)carbonyl or the radical $-CH_2-NH-COO-(C_1-C_2\text{-alkyl})$; and
$R_{12}$ represents hydrogen, $C_1$-$C_6$-alkyl, phenyl substituted by 1 or 2 substituents from the group consisting of halogen, $C_1$-$C_2$-alkyl, $C_2$-$C_4$-alkenyl, $C_2$-$C_3$-alkynyl, $C_1$-$C_2$-alkoxy and $C_2$-$C_3$-alkylthio, or represents pyridyl, furan-2-yl, thien-2-yl or the radical

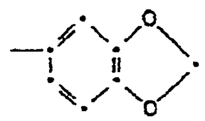

;

or

$R_{12}$ and $R_{11}$ together represent one of the groups $-(CH_2)_3-$, $-(CH_2)_4-$ or $-(CH_2)_5-$.

6. A compound of formula I according to claim 5, characterised in that

$R_1$, $R_2$, $R_3$, $R_4$, $R_5$, $R_6$ and $R_7$ represent hydrogen;

$R_8$ represents fluorine, chlorine or trifluoromethyl;

U represents the grouping -CH=;

X represents -O-;

Y represents -O-, -S-, $-CH_2-$ or -CO-;

n and m are the number 0;

p is a number 1 or 2; and

Z represents the radical

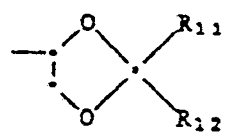

wherein

$R_{11}$ represents hydrogen, $C_1$-$C_6$-alkyl, $C_3$-$C_6$-cycloalkyl, $C_2$-$C_4$-alkenyl, $C_2$-$C_4$-alkynyl, alkoxyalkyl having a total of 2 to 4 carbon atoms, $C_1$-$C_4$-alkyl substituted by 1 to 5 fluorine or chlorine atoms, benzyl, cyanomethyl, ethoxycarbonyl or the radical $-CH_2-NH-COO-CH_3$; and

$R_{12}$ represents hydrogen, $C_1$-$C_6$-alkyl, phenyl substituted by 1 or 2 substituents from the group consisting of fluorine, chlorine, $C_1$-$C_2$-alkyl and $C_1$-$C_2$-alkoxy, or represents pyrid-4-yl, furan-2-yl or thien-2-yl, or the radical

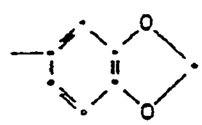

;

or

$R_{12}$ and $R_{11}$ together represent one of the groups $-(CH_2)_3-$, $-(CH_2)_4-$ or $-(CH_2)_5-$.

7. A compound according to claim 4 of formula Ia

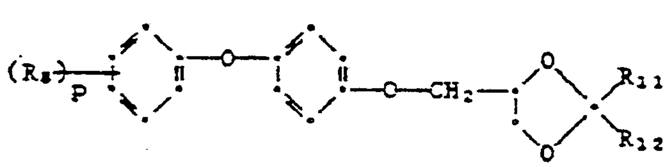

(Ia)

wherein

$R_8$ represents fluorine, chlorine or trifluoromethyl;

p is a number 1 or 2; and

$R_{11}$ and $R_{12}$ each independently of the other represents hydrogen, $C_1$-$C_4$-alkyl, $C_2$-$C_4$-methoxyalkyl, $C_1$-$C_2$-alkyl substituted by 1 to 3 halogen atoms, $C_2$-$C_3$-alkenyl or $C_2$-$C_3$-alkynyl, or $R_{11}$ and $R_{12}$ together represent one of the radicals $-(CH_2)_4-$ or $-(CH_2)_5-$.

8. A compound according to any one of claims 1 to 7, characterised in that p = 1.

9. A compound according to any one of claims 1 to 8, characterised in that $R_{12}$ represents hydrogen.

10. A compound according to claim 9 of formula

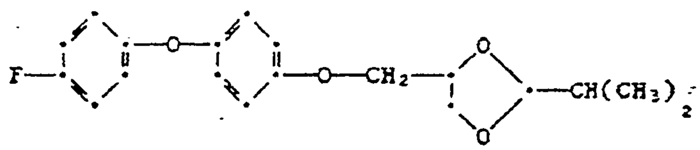

11. A compound according to claim 8 of formula

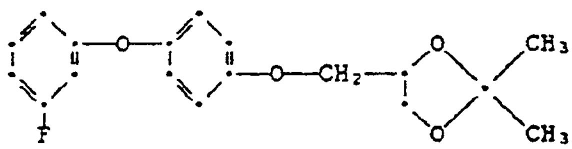

12. A compound according to claim 9 of formula

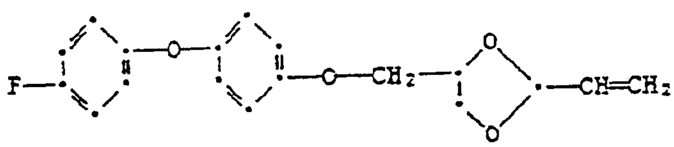

13. A compound according to claim 9 of formula

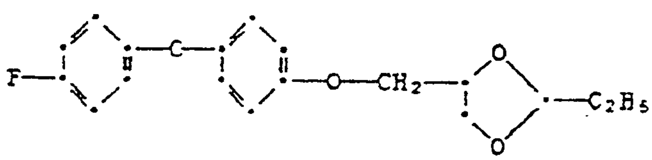

14. A compound according to claim 9 of formula

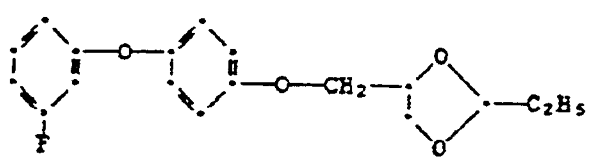

15. A compound according to claim 9 of formula

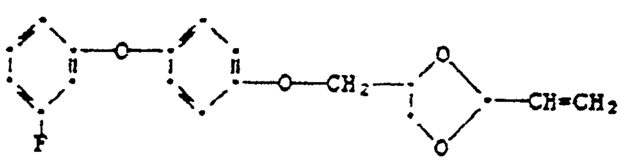

16. A compound according to claim 9 of formula

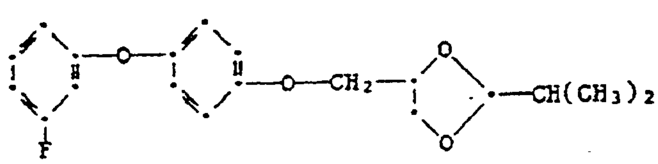

17. A compound according to claim 8 of formula

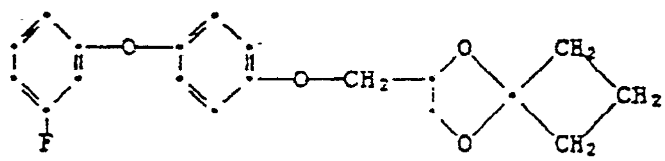

18. A compound according to claim 9 of formula

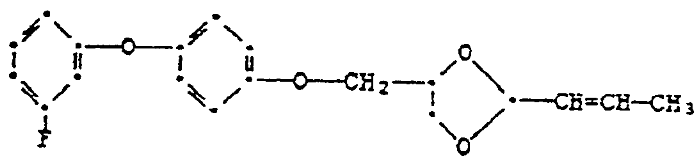

19. A compound according to claim 7 of formula

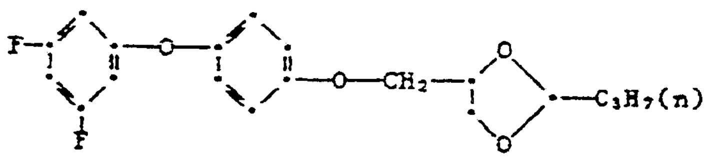

20. A compound according to claim 9 of formula

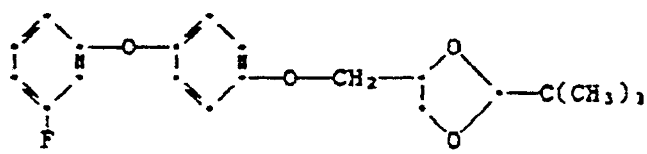

21. A compound according to claim 7 of formula

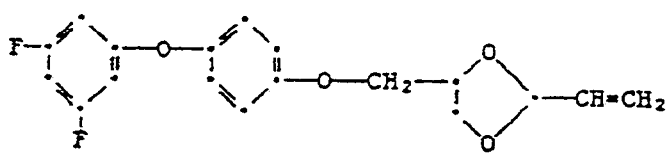

22. A compound according to claim 5 of formula

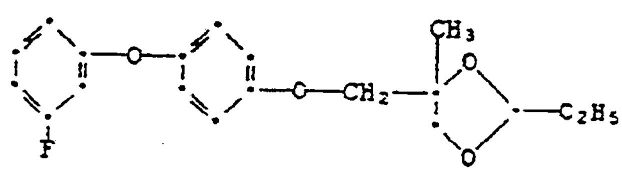

23. A compound according to claim 9 of formula

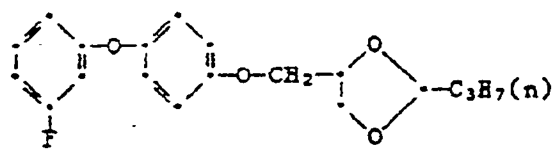

24. A process for the preparation of a compound of formula I according to any one of claims 1 to 23, characterised in that a compound of formula II

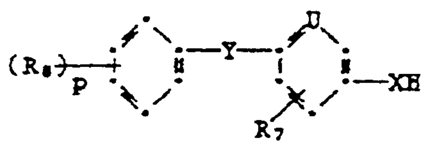 (II)

is reacted with a compound of formula III

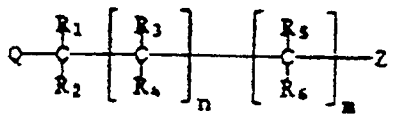 (III),

in which formulae II and III $R_1$ to $R_8$, U, X, Y, n, m, p and Z have the meanings given in claims 1 to 9 and Q represents a leaving group.

25. A process for the preparation of a compound of formula I according to any one of claims 1 to 23 in which Z represents the radical

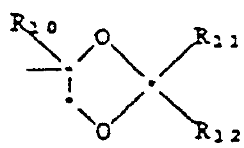 ,

characterised in that a compound of formula IV

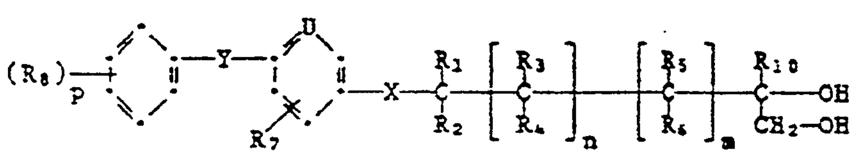 (IV)

is condensed in the presence of an acidic catalyst with a compound of formula V or Va

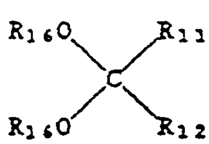 (V) 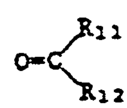 (Va)

in which formulae IV, V and Va $R_1$ to $R_{10}$, U, X, Y, n, m, p, $R_{11}$ and $R_{12}$ have the meanings given in claims 1 to 9 and $R_{16}$ represents methyl or ethyl.

26. A compound of formula IV according to claim 25.

27. A pesticide which contains as active ingredient a compound according to any one of claims 1 to 23 together with suitable carriers and/or other adjuvants.

28. The use of a compound according to any one of claims 1 to 23 for controlling insects and representatives of the order Acarina.

29. The use according to claim 28 for controlling plant-destructive sucking insects.

30. The use according to claim 29 for controlling insects of the family Coccidae.

31. The use according to claim 28 for controlling larval stages of plant-destructive insects.

32. A method for controlling insects and representatives of the order Acarina, characterised in that the pests, or the various stages of development thereof, or their habitat, is/are brought into contact or treated with a pesticidally effective amount of a compound of formula I according to any one of claims 1 to 23 or with a composition containing a pesticidally effective amount of that compound together with adjuvants and carriers.

## Revendications

1. Composé de formule I

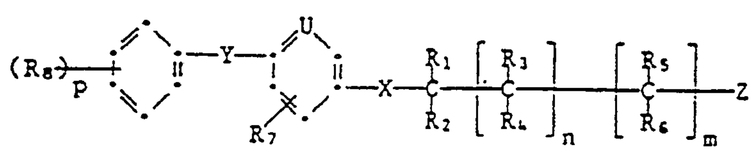 (I),

où

$R_1$, $R_2$, $R_3$, $R_4$, $R_5$ et $R_6$ représentent indépendamment l'un de l'autre un hydrogène ou un alcoyle en $C_{1-4}$ ou, lorsque n = 1,

$R_2$ et $R_4$ représentent ensemble l'un des radicaux $-(CH_2)_3-$ ou $-(CH_2)_4-$ ;

$R_7$ représente un hydrogène, un halogène, un méthyle, un éthyle, un alcoyle en $C_{1-2}$ substitué par de 1 à 5 atomes d'halogène, un méthoxy, un éthoxy ou un alcoxy en $C_{1-2}$ substitué par de 1 à 5 atomes d'halogène ;

$R_8$ représente un halogène, un alcoyle en $C_{1-3}$, un alcoyle en $C_{1-3}$ substitué par de 1 à 7 atomes d'halogène, un alcoxy en $C_{1-3}$, un alcoxy en $C_{1-3}$ substitué par de 1 à 7 atomes d'halogène ou un cyano ;

U représente un groupement -CH= ou -N= ;

X représente -O-, -S- ou $-N(R_9)-$ ;

Y représente -O-, -S-, -S(O)-, $-S(O_2)-$, $-CH_2-$, -CO-, ou $-N(R_9)-$, où

$R_9$ représente un hydrogène, un alcoyle en $C_{1-4}$ ou un alcoylecarbonyle avec un total de 2 à 4 atomes de carbone ;

n représente le nombre 0 ou 1 ;

m représente le nombre 0, 1 ou 2 ;

p représente 1, 2 ou 3 ; et

Z représente le radical

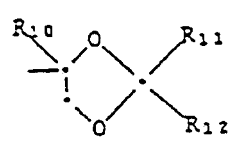

où

$R_{10}$ représente un hydrogène, un méthyle ou un éthyle ;

$R_{11}$ représente un hydrogène, un alcoyle en $C_{1-6}$, cycloalcoyle en $C_{3-6}$, alcényle en $C_{2-4}$, alcynyle en $C_{2-4}$, alcoxyalcoyle avec un total de 2 à 6 atomes de carbone, alcoyle en $C_{1-6}$ substitué par de 1 à 7 atomes de carbone, benzyle, ou benzyle substitué dans son noyau par un atome d'halogène, un radical alcoyle en $C_{1-3}$ ou un radical alcoxy en $C_{1-3}$, un cyanométhyle, (alcoxy en $C_{1-2}$)-carbonyle, ou le radical $-CH_2-NH-COO-$(alcoyle en $C_{1-2}$) ; et

$R_{12}$ représente un hydrogène, alcoyle en $C_{1-6}$, phényle, phényle substitué par de 1 à 3 substituants du groupe halogène, alcoyle en $C_{1-4}$, alcoyle en $C_{1-3}$ substitué par de 1 à 7 atomes d'halogène, alcényle en $C_{2-4}$, alcinyle en $C_{2-4}$, alcoxy en $C_{1-3}$, alcoxy en $C_{1-3}$ substitué par de 1 à 7 atomes d'halogène, alcoxyalcoyle avec un total de 2 à 5 atomes de carbone, alcényloxy en $C_{3-4}$, alcynyloxy en $C_{3-4}$ et alcoylthio en

$C_{1-3}$, un pyridyle, furan-2-yle, thién-2-yle ou le radical

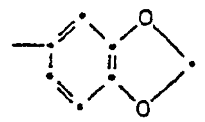 ;

ou

$R_{12}$ et $R_{13}$ représentent ensemble l'un des groupes $-(CH_2)_3-$, $-(CH_2)_4-$ ou $-(CH_2)_5-$

2. Composé de formule I selon la revendication 1, caractérisé en ce que

$R_1$, $R_2$, $R_3$, $R_4$, $R_5$ et $R_6$ représentent indépendamment l'un de l'autre un hydrogène ou un alcoyle en $C_{1-4}$ ou, lorsque n = 1,

$R_2$ et $R_4$ représentent ensemble l'un des radicaux $-(CH_2)_3-$ ou $-(CH_2)_4-$ ;

$R_7$ représente un hydrogène, halogène, méthyle, éthyle, alcoyle en $C_{1-2}$ substitué par de 1 à 5 atomes d'halogène, méthoxy, éthoxy, ou alcoxy en $C_{1-2}$ substitué par de 1 à 5 atomes d'halogène ;

$R_8$ représente un halogène, alcoyle en $C_{1-3}$, alcoyle en $C_{1-3}$ substitué par de 1 à 7 atomes d'halogène, alcoxy en $C_{1-3}$, alcoxy en $C_{1-3}$ substitué par de 1 à 7 atomes d'halogène ou cyano ;

U représente un groupement -CH= ou -N= ;

X représente -O-, -S- ou $-N(R_9)-$ ;

Y représente -O-, -S-, -S(O)-, $-S(O_2)-$, $-CH_2-$, -CO- ou $-N(R_9)-$, où

$R_9$ représente un hydrogène, alcoyle en $C_{1-4}$ ou alcoylecarbonyle avec un total de 2 à 4 atomes de carbone ;

n représente le nombre 0 ou 1 ;

m représente le nombre 0, 1 ou 2 ;

p représente le nombré 1, 2 ou 3 ; et

Z représente le radical

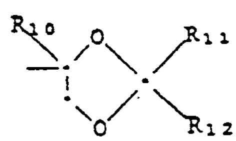

où

$R_{10}$ représente un hydrogène, méthyle ou éthyle ;

$R_{11}$ représente un hydrogène, alcoyle en $C_{1-6}$, cycloalcoyle en $C_{3-6}$, alcényle en $C_{2-4}$, alcynyle en $C_{2-4}$, alcoxyalcoyle avec un total de 2 à 6 atomes de carbone, alcoyle en $C_{1-6}$ substitué par de 1 à 7 atomes d'halogène, benzyle, ou benzyle substitué dans son noyau par un atome d'halogène, un radical alcoyle en $C_{1-3}$ ou un radical alcoxy en $C_{1-3}$ ; et

$R_{12}$ représente un hydrogène, alcoyle en $C_{1-6}$, phényle, un phényle substitué par de 1 à 3 substituants du groupe halogène, alcoyle en $C_{1-4}$, alcoyle en $C_{1-3}$ substitué par de 1 à 7 atomes d'halogène, alcényle en $C_{2-4}$, alcynyle en $C_{2-4}$, alcoxy en $C_{1-3}$, alcoxy en $C_{1-3}$ substitué par de 1 à 7 atomes d'halogène, alcoxyalcoyle avec un total de 2 à 5 atomes de carbone, alcényloxy en $C_{3-4}$, alcynyloxy en $C_{3-4}$ et alcoylthio en $C_{1-3}$, ou le radical

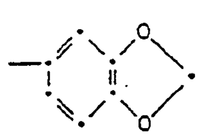 ;

ou

$R_{12}$ et $R_{13}$ représentent ensemble l'un des radicaux $-(CH_2)_4-$ ou $-(CH_2)_5-$

3. Composé de formule I selon l'une des revendications 1 ou 2, caractérisé en ce que
$R_1$ et $R_3$ représentent indépendamment l'un de l'autre un hydrogène, méthyle ou éthyle ;
$R_2$, $R_4$, $R_5$ et $R_6$ représentent un hydrogène ou lorsque n = 1,
$R_2$ et $R_4$ représentent ensemble l'un des radicaux $-(CH_2)_3-$ ou $-(CH_2)_4-$ ;
$R_7$ représente un hydrogène ou un halogène ;
$R_8$ représente un méthyle, éthyle, halogène ou alcoyle en $C_{1-2}$ substitué par de 1 à 5 atomes d'halogène ;
U représente un groupement -CH= ou -N= ;
X représente -O-, -S- ou -N($R_9$)- ;
Y représente -O-, -S-, $-CH_2-$ ou -CO- ; où
$R_9$ représente un hydrogène, méthyle ou acétyle ;
n et m représentent indépendamment l'un de l'autre le nombre 0 ou 1 ; et
p représente le nombre 1 ou 2 ; et
Z représente le radical

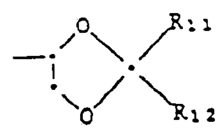

où
$R_{11}$ représente un hydrogène, alcoyle en $C_{1-4}$, cycloalcoyle en $C_{3-6}$, alcényle en $C_{2-3}$, alcynyle en $C_{2-3}$, alcoxyalcoyle avec au total de 2 à 4 atomes de carbone, alcoyle en $C_{1-4}$ substitué par de 1 à 7 atomes d'halogène ou benzyle ; et
$R_{12}$ représente un hydrogène, alcoyle en $C_{1-4}$, phényle, phényle substitué par un ou deux substituants du groupe halogène, alcoyle en $C_{1-3}$, alcoyle en $C_{1-2}$ substitué par de 1 à 5 atomes d'halogène, alcényle en $C_{2-4}$, alcynyle en $C_{2-4}$, alcoxy en $C_{1-3}$, méthoxy substitué par de 1 à 3 atomes d'halogène, alcoxyalcoyle avec au total de 3 à 4 atomes de carbone, alcényloxy en $C_{3-4}$, alcynyloxy en $C_{3-4}$ et alcoylthio en $C_{1-3}$ ou le radical

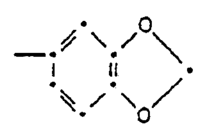 ;

ou

$R_{12}$ et $R_{11}$ représentent ensemble l'un des radicaux $-(CH_2)_4-$ ou $-(CH_2)_5-$

4. Composé de formule I, selon la revendication 3, caractérisé en ce que
$R_1$ représente un hydrogène ou un méthyle ;
$R_2$, $R_3$, $R_4$, $R_5$, $R_5$ et $R_7$ représentent un hydrogène ;
$R_8$ représente un fluor, un chlore, un éthyle, ou un trifluorométhyle ;
U représente le groupement -CH= ;
X représente -O- ;
Y représente -O- ;
n et m représentent le nombre 0 ;
p représente le nombre 1 ou 2 ; et
Z représente le radical

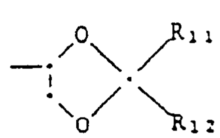

où

$R_{11}$ représente un hydrogène, alcoyle en $C_{1-4}$, cycloalcoyle en $C_{3-6}$, alcényle en $C_{2-3}$, alcynyle en $C_{2-3}$, alcoxyalcoyle avec au total de 2 à 4 atomes de carbone, ou benzyle ;

$R_{12}$ représente un hydrogène, alcoyle en $C_{1-4}$, alcoyle en $C_{1-2}$ substitué par de 1 à 5 atomes d'halogène, phényle, phényle substitué par un ou deux substituants du groupe halogène et alcoyle en $C_{1-3}$, ou le radical

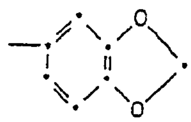 ;

ou

$R_{12}$ et $R_{11}$ représentent ensemble l'un des radicaux $-(CH_2)_4-$ ou $-(CH_2)_5-$

5. Composé de formule I, selon la revendication 1, caractérisé en ce que

$R_1$ représente un hydrogène ou un méthyle ;

$R_2$, $R_3$, $R_4$, $R_5$, $R_6$ et $R_7$ représentent un hydrogène ;

$R_8$ représente un halogène ou un trifluorométhyle ;

U représente le groupement -CH= ;

X représente -O- ou -S- ;

Y représente -O-, -S-, $-CH_2-$ ou -CO- ;

n et m représentent le nombre 0 ;

p représente le nombre 1 ou 2 ; et

Z représente le radical

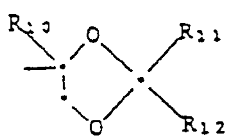

où

$R_{10}$ représente un hydrogène ou un méthyle ;

$R_{11}$ représente un hydrogène, alcoyle en $C_{1-6}$, cycloalcoyle en $C_{3-6}$, alcényle en $C_{2-4}$, alcynyle en $C_{2-4}$, alcoxyalcoyle avec au total de 2 à 4 atomes de carbone, alcoyle en $C_{1-4}$ substitué par de 1 à 5 atomes d'halogène, benzyle, cyanométhyle, (alcoxy en $C_{1-2}$)-carbonyle, ou le radical $-CH_2$-NH-COO-(alcoyle en $C_{1-2}$) ; et

$R_{12}$ représente un hydrogène, alcoyle en $C_{1-6}$, phényle substitué par un à deux substituants du groupe halogène, alcoyle en $C_{1-2}$, alcényle en $C_{2-4}$, alcynyle en $C_{2-3}$, alcoxy en $C_{1-2}$, et alcoylthio en $C_{2-3}$, pyridyle, furan-2-yle, thièn-2-yle, ou le radical

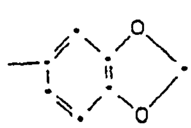 ;

ou

$R_{12}$ et $R_{11}$ représentent ensemble l'un des groupes $-(CH_2)_3-$, $-(CH_2)_4-$ ou $-(CH_2)_5-$

6. Composé de formule I selon la revendication 5, caractérisé en ce que

$R_1$, $R_2$, $R_3$, $R_4$, $R_5$, $R_6$ et $R_7$ représentent un hydrogène ;

$R_8$ représente un fluor, chlore ou trifluorométhyle ;

U représente le groupement -CH= ;

X représente -O- ;

Y représente -O-, -S-, $-CH_2-$ ou -CO- ;
n et m représentent le nombre 0 ;
p représente le nombre 1 ou 2 ; et
Z représente le radical

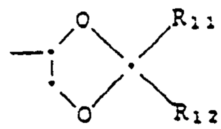

où

$R_{11}$ représente un hydrogène, alcoyle en $C_{1-6}$, cycloalcoyle en $C_{3-6}$, alcényle en $C_{2-4}$, alcynyle en $C_{2-4}$, alcoxyalcoyle avec au total de 2 à atomes de carbone, alcoyle en $C_{1-4}$ substitué par de 1 à 5 atomes de fluor ou de chlore, benzyle, cyanométhyle, éthoxycarbonyle ou le radical $-CH_2-NH-COO-CH_3$ ; et
$R_{12}$ représente un hydrogène, alcoyle en $C_{1-6}$, phényle substitué par de 1 à 2 substituants du groupe fluor, chlore; alcoyle en $C_{1-2}$ et alcoxy en $C_{1-2}$, pyrid-4-yle, furan-2-yle, ou thièn-2-yle ou le radical

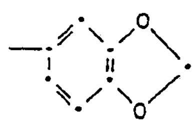 ;

ou

$R_{12}$ et $R_{11}$ représentent ensemble l'un des groupes $-(CH_2)_3-$, $-(CH_2)_4-$ ou $-(CH_2)_5-$

7. Composé selon la revendication 4, de formule Ia

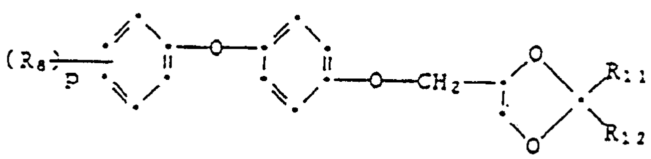 (Ia),

où

$R_8$ représente un fluor, un chlore ou-un trifluorométhyle ;
p représente le nombre 1 ou 2 ; et
$R_{11}$ et $R_{12}$représentent indépendamment l'un de l'autre un hydrogène, alcoyle en $C_{1-4}$, un méthoxyalcoyle en $C_{2-4}$, alcoyle en $C_{1-2}$ substitué par de 1 à 3 atomes d'halogène, alcényle en $C_{2-3}$ ou alcynyle en $C_{2-3}$ ou

$R_{11}$ et $R_{12}$ représentent ensemble l'un des radicaux $-(CH_2)_4-$ ou $-(CH_2)_5-$

8. Composé selon l'une des revendications 1 à 7, caractérisé en ce que p = 1.

9. Composé selon l'une quelconque des revendications 1 à 8, caractérisé en ce que $R_{12}$ représente un hydrogène.

10. Composé selon la revendication 9, de formule

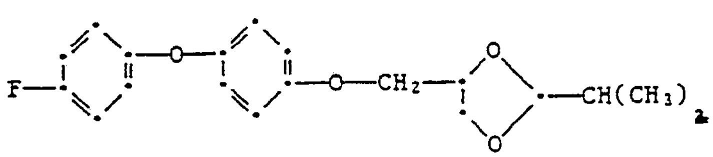

11. Composé selon la revendication 8, de formule

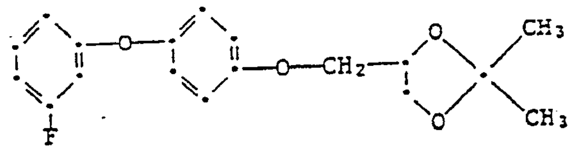

12. Composé selon la revendication 9, de formule

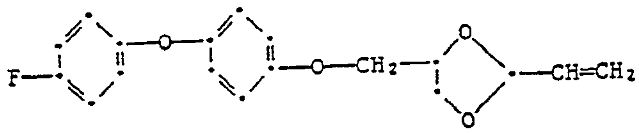

13. Composé selon la revendication 9, de formule

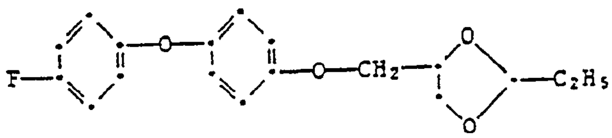

14. Composé selon la revendication 9, de formule

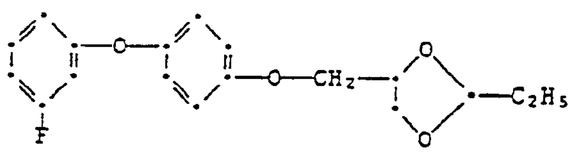

15. Composé selon la revendication 9, de formule

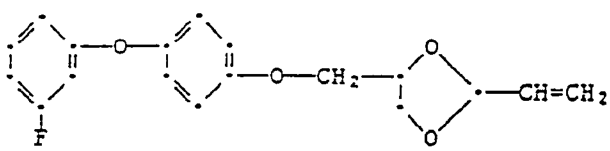

16. Composé selon la revendication 9, de formule

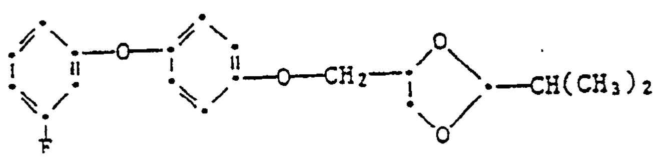

17. Composé selon la revendication 8, de formule

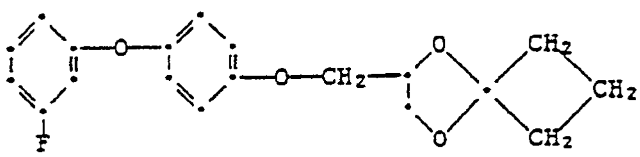

18. Composé selon la revendication 9, de formule

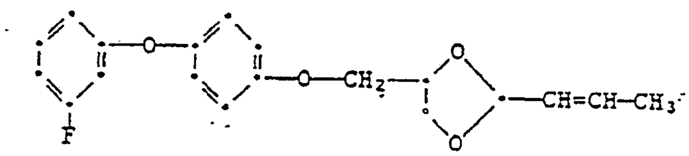

19. Composé selon la revendication 7, de formule

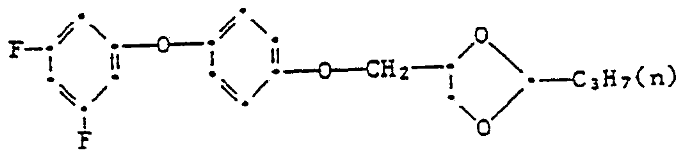

20. Composé selon la revendication 9, de formule

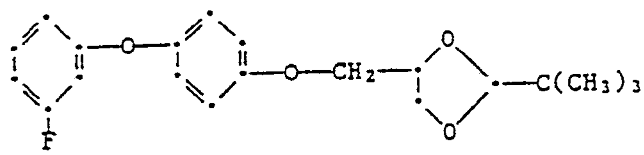

21. Composé selon la revendication 7, de formule

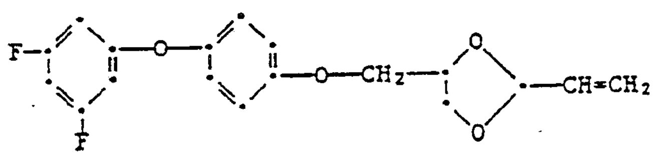

22. Composé selon la revendication 5, de formule

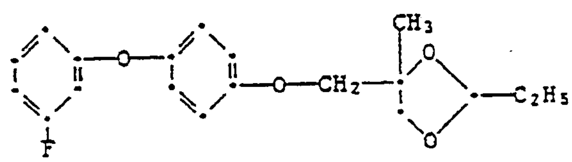

23. Composé selon la revendication 9, de formule

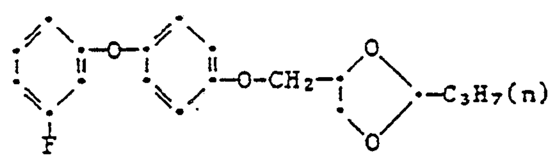

24. Procédé de préparation d'un composé de formule I selon l'une des revendications 1 à 23, caractérisé en ce qu'on fait réagir un composé de formule II

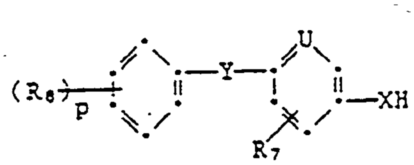

(II)

avec un composé de formule III

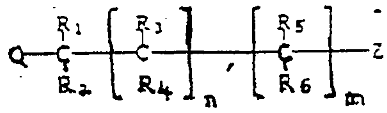

(III)

où dans les formules II et III $R_1$ à $R_8$, U, X, Y, n, m, p, et Z ont les significations données dans les revendications 1 à 9 et Q représente un groupe sortant.

25. Procédé de préparation d'un composé de formule I selon l'une des revendications 1 à 23, où Z représente le radical

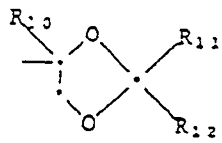

caractérisé en ce qu'on condense un composé de formule IV

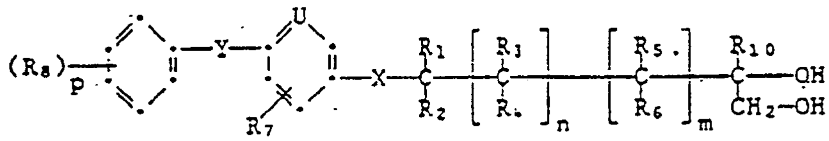 (IV)

en présence d'un catalyseur acide avec un composé de formule V ou Va

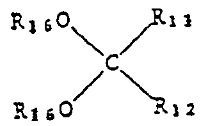 (V) 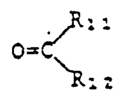 (Va)

où dans les formules IV, V et Va, $R_1$ à $R_{10}$, U, X, Y, n, m, p, $R_{11}$ et $R_{12}$ ont les significations données dans les revendications 1 à 9, et $R_{16}$ représente un méthyle ou un éthyle.

26. Composé de formule IV selon la revendication 25.

27. Agent de lutte antiparasitaire qui contient comme composant actif un composé selon l'une des revendications 1 à 23, avec des supports et/ou autres additifs appropriés.

28. Application d'un composé selon l'une quelconque des revendications 1 à 23, pour combattre les insectes et les représentants de l'ordre des Acariens.

29. Application d'un composé selon la revendication 28 pour combattre les insectes suceurs causant des dommages aux plantes.

30. Application selon la revendication 29, pour combattre les cochenilles.

31. Application selon la revendication 28, pour combattre les stades larvaires des insectes qui causent des dommages aux plantes.

32. procédé de lutte contre les insectes et représentants de l'ordre des Acariens, caractérisé en ce qu'on met,en contact ou en ce qu'on traite les parasites ou leurs différents stades de développement, ou leur habitat avec une quantité à action pesticide d'un composé de formule I selon l'une des revendications 1 à 23 ou avec un agent contenant, outre des additifs, et des supports, une quantité à action pesticide de ce composé.